# EUROPEAN PATENT APPLICATION

(11) **EP 4 749 543 A1**
(43) Date of publication of application: **27.05.2026**
(21) Application number: 25212826.9
(22) Date of filing: 31.10.2025
(51) Int. Cl.: G06Q 10/0633, G16H 10/40

(54) **LARGE LANGUAGE MODEL RESOLUTION OF SCIENTIFIC INSTRUMENT WORKFLOW INTERRUPTIONS**

(30) Priority: 20.11.2024 US 202418953820
(71) Applicant: FEI COMPANY, Hillsboro, OR 97124 (US)
(72) Inventor: VÍTECEK, Jirí, Boleradice (CZ); MACHEK, Ondrej, Brno (CZ); JANCÍK, Radek, Brno (CZ)
(74) Representative: Boult Wade Tennant LLP

(57) **Abstract**

Systems/techniques are provided for facilitating large language model resolution of scientific instrument workflow interruptions. In various embodiments, a system can cause a scientific instrument (e.g., charged-particle microscope, chromatograph, mass-spectrometer) to perform a workflow on a specimen. In various aspects, the system can, in response to the scientific instrument generating an error message that interrupts the workflow, retrieve runtime data logged by the scientific instrument during the workflow. In various instances, the system can synthesize, via execution of a large language model on the error message and on the runtime data, first text that explains why the workflow was interrupted.

## Description

### BACKGROUND

The technical field of scientific instruments has been historically constrained by its operational complexity. Such operational complexity can impede efficient troubleshooting of errors experienced by scientific instruments.

### SUMMARY

The following presents a summary to provide a basic understanding of one or more embodiments. This summary is not intended to identify key or critical elements, or delineate any scope of the particular embodiments or any scope of the claims. Its sole purpose is to present concepts in a simplified form as a prelude to the more detailed description that is presented later. In one or more embodiments described herein, devices, systems, computer-implemented methods, apparatus or computer program products that facilitate large language model resolution of scientific instrument workflow interruptions are described.

According to one or more embodiments, a system is provided. The system can comprise a non-transitory computer-readable memory that can store computer-executable components. The system can further comprise a processor that can be operably coupled to the non-transitory computer-readable memory and that can execute the computer-executable components stored in the non-transitory computer-readable memory. In various embodiments, the computer-executable components can comprise a workflow component that can cause a charged-particle microscope to perform a workflow on a specimen. In various aspects, the computer-executable components can comprise a status component that, in response to the charged-particle microscope generating an error message that interrupts the workflow, can retrieve runtime data logged by the charged-particle microscope during the workflow. In various instances, the computer-executable components can comprise a model component that can synthesize, via execution of a large language model on the error message and on the runtime data, first text that explains why the workflow was interrupted.

According to one or more embodiments, a computer-implemented method is provided. In various embodiments, the computer-implemented method can comprise causing, by a device operatively coupled to a processor, a charged-particle microscope to perform a workflow on a specimen. In various aspects, the computer-implemented method can comprise retrieving, by the device and in response to the charged-particle microscope generating an error message that interrupts the workflow, runtime data logged by the charged-particle microscope during the workflow. In various instances, the computer-implemented method can comprise synthesizing, by the device and via execution of a large language model on the error message and on the runtime data, first text that explains why the workflow was interrupted.

According to one or more embodiments, a computer program product for facilitating large language model resolution of scientific instrument workflow interruptions is provided. In various embodiments, the computer program product can comprise a non-transitory computer-readable memory having program instructions embodied therewith. In various aspects, the program instructions can be executable by a processor to cause the processor to cause a scientific instrument to perform a workflow on a specimen. In various instances, the program instructions can be executable by the processor to cause the processor to retrieve, in response to the scientific instrument generating an error message that interrupts the workflow, runtime data logged by the scientific instrument during the workflow. In various cases, the program instructions can be executable by the processor to cause the processor to synthesize, via execution of a large language model on the error message and on the runtime data, first text that explains why the workflow was interrupted. In various aspects, the scientific instrument can be a charged-particle microscope, a chromatograph, or a mass spectrometer.

### DESCRIPTION OF THE DRAWINGS

Various embodiments will be readily understood by the following detailed description in conjunction with the accompanying figures. To facilitate this description, like reference numerals designate like structural elements. Embodiments are illustrated by way of example, not by way of limitation, in the figures. The figures are not necessarily drawn to scale.
FIG. 1 illustrates an example, non-limiting block diagram of a scientific instrument module in accordance with various embodiments described herein.
FIG. 2 illustrates an example, non-limiting flow diagram of a computer-implemented method in accordance with various embodiments described herein.
FIG. 3 illustrates a block diagram of an example, non-limiting system that facilitates large language model resolution of scientific instrument workflow interruptions in accordance with one or more embodiments described herein.
FIG. 4 illustrates a block diagram of an example, non-limiting system including an instrument workflow and an error message that facilitates large language model resolution of scientific instrument workflow interruptions in accordance with one or more embodiments described herein.
FIG. 5 illustrates an example, non-limiting block diagram showing how an error message can interrupt an instrument workflow in accordance with one or more embodiments described herein.
FIG. 6 illustrates a block diagram of an example, non-limiting system including instrument runtime data that facilitates large language model resolution of scientific instrument workflow interruptions in accordance with one or more embodiments described herein.
FIG. 7 illustrates an example, non-limiting block diagram of instrument runtime data in accordance with one or more embodiments described herein.
FIG. 8 illustrates a block diagram of an example, non-limiting system including a technical document repository and a solution that facilitates large language model resolution of scientific instrument workflow interruptions in accordance with one or more embodiments described herein.
FIGs. 9-14 illustrate example, non-limiting block diagrams showing how a solution to an error message can be generated based on instrument runtime data and a technical document repository in accordance with one or more embodiments described herein.
FIG. 15 illustrates a block diagram of an example, non-limiting system including solution feedback and a set of technical document edits that facilitates large language model resolution of scientific instrument workflow interruptions in accordance with one or more embodiments described herein.
FIG. 16 illustrates an example, non-limiting block diagram showing how one or more technical document edits can be generated based on solution feedback in accordance with one or more embodiments described herein.
FIG. 17 illustrates an example, non-limiting block diagram showing how a large language model can be trained in accordance with one or more embodiments described herein.
FIG. 18 illustrates a block diagram of an example, non-limiting operating environment in which one or more embodiments described herein can be facilitated.
FIG. 19 illustrates an example networking environment operable to execute various implementations described herein.
FIG. 20 illustrates an example dual beam microscope that can be implemented in accordance with various embodiments described herein.

### DETAILED DESCRIPTION

The following detailed description is merely illustrative and is not intended to limit embodiments or application/uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Various operations can be described as multiple discrete actions or operations in turn, in a manner that is most helpful in understanding the subject matter disclosed herein. However, the order of description should not be construed as to imply that these operations are necessarily order dependent. In particular, these operations can be performed in an order different from the order of presentation. Operations described can be performed in a different order from the described embodiments. Various additional operations can be performed, or described operations can be omitted in additional embodiments.

Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices. As used herein, the phrase "based on" should be understood to mean "based at least in part on," unless otherwise specified.

Scientific instruments are highly-complex combinations of hardware and software that facilitate high-precision measurements of physical specimens (e.g., semiconductor wafers, lamellas, aqueous mixtures, biological tissues) in scientific, laboratory, research, or clinical operational environments by leveraging complicated arrangements of actuatable parts (e.g., ion sources, electron sources, optical lenses or apertures, optical plates or deflectors, columns, coils, heaters, coolers, fluid valves, fluid pumps, circuit switches, specimen stages), sensors (e.g., ion detectors, electron detectors, voltmeters, thermistors, potentiometers, pressure gauges), or consumables (e.g., carrier fluids, calibrants, filters, reactive gases). For instance, a charged-particle microscope (e.g., a scanning electron microscope (SEM), a transmission electron microscope (TEM), an electron energy-loss microscope (EELM)) is a type of scientific instrument that can capture or generate microscopic or nanoscopic images or energy spectra of physical specimens. As another instance, a mass spectrometer, which may be coupled to or outfitted with a chromatograph, is another type of scientific instrument that can capture or generate ion abundance data (e.g., chromatograms, mass spectra) associated with the chemical or molecular composition of a physical specimen.

The technical field of scientific instrumentation has been historically constrained by its operational complexity. In other words, because scientific instruments can have such complicated constructions, they can be commensurately complicated to operate or use. Indeed, in order for a user to competently or confidently use a scientific instrument to analyze clinical or laboratory specimens, that user often requires extensive specialized training, education, or certification with respect to the scientific instrument. For example, the user can learn how to properly operate a graphical user-interface (GUI) or physical controls of a charged-particle microscope or a mass spectrometer by studying weeks-long or months-long instrumentation courses. After all, a charged-particle microscope or a mass spectrometer can, at first glance, have a dizzying or overwhelming number of configurable software or hardware settings, buttons, knobs, sliders, or options. A user that does not undergo such extensive studying can be unable to competently use the charged-particle microscope or the mass spectrometer. For example, a user that does not take an appropriate weeks-long or months-long instrumentation course, but that nevertheless attempts to operate the charged-particle microscope or the mass spectrometer, can be significantly likely to damage specimens or the instruments themselves.

This need for extensive study or skill can be significantly compounded in view of the fact that different types of scientific instruments can function or be operated differently than each other. That is, specialized instrumentation training, education, or certification is often not transferrable between, among, or across different types of scientific instruments. For example, whatever training that readies one to competently operate an SEM does not necessarily ready one to competently operate a TEM and certainly does not ready one to competently operate a mass spectrometer. As another example, whatever training that readies one to competently operate an SEM having model number A does not necessarily ready one to competently operate an SEM having model number B. As even another example, whatever training that readies one to competently operate an SEM having model number A and software version C does not necessarily ready one to competently operate an SEM having model number A and software version D.

Because of such immense operational complexity, users of scientific instruments often experience and are unable to resolve hardware-related or software-related malfunctions that arise during operation of such scientific instruments. To troubleshoot or otherwise deal with such malfunctions, manufacturers of scientific instruments often offer technical support services to users. For example, the manufacturer can employ numerous field engineers, technical specialists, or research- and-development scientists to interact with users, with the goal or purpose of troubleshooting whatever instrumentation malfunctions those users encounter. Depending upon the severity or extent of any given malfunction (e.g., the given malfunction might be a simple, commonly-occurring issue; or the given malfunction might instead be a never-before-seen software glitch or a never-before-seen hardware failure), those technical support services can consume excessive amounts of time (e.g., hours, days, or even weeks) to evaluate the given malfunction, to brainstorm solutions to the given malfunction, and to actually implement or enact those solutions so as to resolve the given malfunction. These difficulties can scale exponentially as the number of scientific instruments provided by the manufacturer to different users grows.

Accordingly, systems or techniques that can reduce the amount of time needed to troubleshoot or resolve malfunctions that may arise during operation of scientific instruments can be desirable.

Various embodiments described herein can address this technical problem. One or more embodiments described herein can include systems, computer-implemented methods, apparatus, or computer program products that can facilitate large language model resolution of scientific instrument workflow interruptions. In other words, various embodiments described herein can leverage large language models (LLMs), such as ChatGPT, to provide fast and effective troubleshooting of scientific instrument malfunctions. Indeed, when various embodiments described herein are implemented, errors, failures, or other malfunctions that interrupt scientific instrument workflows can be resolved in mere second or minutes, as opposed to hours, days, or weeks. Thus, users of such scientific instruments need not wait excessively long periods of time for troubleshooting services or repairs to be provided, brainstormed, or developed by field engineers, technical specialists, or research scientists. Instead, an LLM can, as described herein, automatically provide, brainstorm, or develop such troubleshooting services or repairs in real-time, which can be considered as desirable, beneficial, or advantageous.

The present inventors devised various embodiments for reducing the amount of time consumed via scientific instrument troubleshooting. As described herein, such reduced time-consumption can be achieved by implementation of: malfunction-triggered LLM resolution synthesis; or feedback-conditioned LLM editing of resolution references.

First, consider malfunction-triggered LLM resolution synthesis. In various embodiments, a scientific instrument can be loaded with a given specimen and can initiate or begin to perform a given workflow on that given specimen (e.g., an imaging workflow, an injection workflow, a milling workflow, a spatial repositioning workflow, a thermal adjustment workflow). In various aspects, the given workflow can be interrupted by a particular malfunction. For example, the scientific instrument can throw or present one or more error codes that cease or pause the workflow. It can be desired to quickly diagnose, troubleshoot, or resolve the particular malfunction, but obtaining such diagnosis, troubleshooting, or resolution from field engineers, technical specialists, or research scientists associated with a manufacturer of the scientific instrument can be highly time-consuming (e.g., can take as long as weeks, depending upon how complicated the particular malfunction is). Accordingly, the scientific instrument can, in various aspects, respond to the particular malfunction by extracting or recalling its runtime data that is associated with the given workflow (e.g., by extracting or recalling whatever images or mass spectra that were partially-captured by the scientific instrument during the given workflow, by extracting or recalling whatever values the controllable operating parameters of the scientific instrument were set to during the given workflow, or by extracting or recalling any other data captured by any other sensors of the scientific instrument during the given workflow). In various cases, the runtime data and the error codes thrown by the scientific instrument can be fed together as a collective input prompt to an LLM, thereby causing the LLM to produce various pieces of synthesized textual content regarding the particular malfunction. Some of that synthesized textual content can be one or more natural language sentences that describe or explain what seems to have caused the scientific instrument to experience the particular malfunction. Other portions of the synthesized textual content can be one or more natural language sentences that describe or explain how to resolve, remedy, or rectify the particular malfunction. Yet other portions of the synthesized textual content can be one or more lines of computer-executable code which, upon execution, can actually implement whatever software-related steps, actions, or modifications would resolve, remedy, or rectify the particular malfunction. In other words, the error codes and the runtime data produced by the scientific instrument can be considered as conveying (possibly in hidden, uninterpretable, or otherwise obscure fashion) at least some amount of measured or quantifiable information that is unique to or characteristic of the particular malfunction, and the LLM can be considered as leveraging such information so as to predict or infer how to resolve the particular malfunction. In some embodiments, the error codes and runtime data can be enhanced in retrieval-augmented generative (RAG) fashion by relevant technical documents that are associated with the scientific instrument (e.g., by operating manuals of the scientific instrument, by design blueprints of the scientific instrument, by coding scripts or files associated with the scientific instrument). In any case, the synthesized textual content can be considered as teaching or explaining why the particular malfunction is occurring or otherwise how to stop the particular malfunction from occurring, and the LLM can consume on the order of mere seconds to generate such synthesized textual content. Thus, triggering the LLM to synthesize a troubleshooting resolution to the particular malfunction in response to the particular malfunction interrupting the given workflow as described herein can help to ensure that the particular malfunction is resolved expeditiously. Contrast this with the days or weeks of troubleshooting that field engineers, technical specialists, or research scientists normally spend attempting to resolve complicated malfunctions that afflict their scientific instruments.

Next, consider feedback-conditioned LLM editing of resolution references. As mentioned above, the LLM can, in response to the given workflow being interrupted by the particular malfunction, generate synthesized textual content that explains or describes why (in the opinion of the LLM) the particular malfunction occurred or how (again, in the opinion of the LLM) to resolve the particular malfunction. As also mentioned above, such synthesized textual content can be based on one or more relevant technical documents that are associated with the scientific instrument and that are treated as RAG references for the LLM. Now, in various aspects, a field engineer, technical specialist, or research scientist can provide feedback regarding the synthesized textual content. In various instances, that feedback can be natural language text written by the field engineer, technical specialist, or research scientist that critiques or otherwise identifies shortcomings of or inaccuracies in the synthesized textual content. In response, that feedback, the synthesized textual content, and the RAG references that the LLM relied upon to generate the synthesized textual content can all be fed together as a collective input prompt to an LLM, thereby causing the LLM to produce additional pieces of synthesized textual content. In various cases, those additional pieces of synthesized textual content can be one or more edits (e.g., word deletions, word insertions) which, if those edits had been incorporated into respective ones of the RAG references prior to generation of the synthesized textual content, would have caused the synthesized textual content to lack or otherwise not have the shortcomings or inaccuracies specified in the feedback. In other words, the feedback can be considered as pinpointing specific inaccuracies in the LLM's synthesized troubleshooting resolution to the particular malfunction, and the LLM can leverage such feedback to: determine which portions of which RAG references caused the LLM to create those specific inaccuracies; and synthesize edits to those portions of those RAG references, so as to help prevent the LLM from creating those same specific inaccuracies in the future. By causing the LLM to infer how its RAG references should be edited so as to avoid whatever resolution shortcomings are identified in the feedback, the LLM can become more likely to provide accurate resolution inferences when responding to future instrument malfunctions. In some cases, this can be referred to as inverted-RAG (e.g., RAG can involve utilizing relevant textual references to synthesize textual content; in contrast, inverted-RAG can involve utilizing feedback regarding synthesized textual content to edit relevant textual references).

Various embodiments described herein can be considered as a computerized tool (e.g., any suitable combination of computer-executable hardware or computer-executable software) that can facilitate large language model resolution of scientific instrument workflow interruptions. In various aspects, such computerized tool can comprise a workflow component, a status component, a model component, or an execution component.

In various embodiments, there can be a scientific instrument. In some cases, the scientific instrument can be a charged-particle microscope exhibiting any suitable design or construction (e.g., can be an SEM, can be a TEM, can be an EELM, can be a dual-beam microscope). In other cases, the scientific instrument can be a chromatograph-equipped mass spectrometer exhibiting any suitable design or construction (e.g., liquid chromatography hardware, gas chromatography hardware, ion chromatography hardware, matrix assisted laser desorption/ionization (MALDI) hardware, electrospray ionization (ESI) hardware, quadrupole mass filter analyzer hardware, ion trap analyzer hardware, time-of-flight (TOF) analyzer hardware, electrostatic trap hardware). In various instances, the scientific instrument can comprise any suitable number of configurable operating settings. In various cases, a configurable operating setting can be any suitable selectively-controllable hardware characteristic or selectively-controllable software characteristic of the scientific instrument that can be directly adjusted or changed in response to electronic instructions or commands received from a user of the scientific instrument (e.g., can be a user-controlled voltage or current setting of the scientific instrument, a user-controlled temperature setting of the scientific instrument, or a user-controlled actuator setting of the scientific instrument). In various aspects, there can be any suitable specimen (e.g., semiconductor wafer or lamella for charged-particle microscopes; aqueous mixture for mass spectrometers) that is currently loaded in the scientific instrument (e.g., that is currently located or positioned on an actuatable stage of a charged-particle microscope; that is currently inside of an autosampler injector of a chromatograph-equipped mass spectrometer).

In various embodiments, there can be an LLM. In various aspects, the LLM can exhibit any suitable deep learning internal architecture. For example, the LLM can include any suitable numbers of any suitable types of layers (e.g., input layer, one or more hidden layers, output layer, any of which can be convolutional layers, dense layers, long short-term memory (LSTM) layers, transformer layers, non-linearity layers, pooling layers, batch normalization layers, or padding layers). As another example, the LLM can include any suitable numbers of neurons in various layers (e.g., different layers can have the same or different numbers of neurons as each other). As yet another example, the LLM can include any suitable activation functions (e.g., softmax, sigmoid, hyperbolic tangent, rectified linear unit) in various neurons (e.g., different neurons can have the same or different activation functions as each other). As still another example, the LLM can include any suitable interneuron connections or interlayer connections (e.g., forward connections, skip connections, recurrent connections).

Regardless of its specific internal architecture, the LLM can be configured as a generative text-to-text model. That is, the LLM can be configured to receive as input any suitable textual data (which, in various cases, may or may not be accompanied by any suitable numerical data or any suitable graphical data), and the LLM can be configured to produce as output synthesized textual content (e.g., one or more synthesized sentences or sentence fragments) that is semantically or substantively based on such inputted textual data (and based on accompanying numerical or graphical data, as the case may be).

In order to accomplish this, the LLM can be considered as comprising an encoder portion and a synthesizer portion. In various aspects, the encoder portion can be any suitable upstream layers of the LLM that are configured to receive the inputted textual data (and any accompanying numerical or graphical data) and to produce embeddings based on that inputted textual data. In various instances, the synthesizer portion can be any suitable downstream layers of the LLM that are configured to receive those embeddings and to produce the synthesized textual content based on those embeddings.

In various aspects, an embedding produced by the encoder portion of the LLM in response to a piece of inputted textual, numerical, or graphical data can be considered as any suitable mathematical quantity (e.g., scalar, vector, matrix, tensor, tokenization, or any suitable combination thereof) that numerically represents at least some substantive or semantic aspect of that inputted textual, numerical, or graphical data in a low-dimensional fashion. In other words, the embedding can be smaller in terms of size or dimensionality (e.g., in some cases, one or more orders of magnitude smaller) than such inputted textual, numerical, or graphical data; but despite such smaller size, the embedding can nevertheless be considered as substantively or semantically representing such inputted textual, numerical, or graphical data. In still other words, the embedding can be considered as a latent vector representation of such inputted textual, numerical, or graphical data.

In any case, it can be desired to leverage the LLM so as to troubleshoot or resolve malfunctions that the scientific instrument experiences. In various instances, the computerized tool described herein can accomplish this.

In various embodiments, the computerized tool can electronically access the LLM or the scientific instrument. For instance, the computerized tool can electronically interface or communicate with (e.g., send electronic commands to, read electronic signals from) the LLM or the scientific instrument. Accordingly, any components of the computerized tool can electronically interact with (e.g., read, write, edit, copy, manipulate, execute, activate, deactivate, modify) the LLM or the scientific instrument.

In various aspects, the workflow component of the computerized tool can electronically cause the scientific instrument to initiate or begin an instrument workflow on whatever specimen is currently loaded in the scientific instrument. In various aspects, the instrument workflow can be any suitable sequence of one or more hardware or software operations that are performable by the scientific instrument. For example, suppose that the scientific instrument is a charged-particle microscope. In such case, the instrument workflow can be: an imaging protocol that is configured or intended to capture microscopic or nanoscopic images of the specimen; a milling protocol that is configured or intended to mill or remove some amount of material from specified locations of the specimen; a stage-actuation protocol that is configured or intended to physically move or reorient the specimen in space; or a vacuum protocol that is configured or intended to pump or vent a vacuum chamber in which the specimen is located to a particular level of pressure. As another example, suppose that the scientific instrument is a chromatograph-equipped mass spectrometer. In such case, the instrument workflow can be: a scanning or screening protocol that is configured or intended to capture chromatograms or mass spectra of the specimen; a flushing protocol (in situations where the specimen is a cleaning mixture) that is configured to flush various channels or conduits of the mass spectrometer with the specimen; or a temperature adjustment protocol that is configured or intended to raise or lower the temperatures of various constituent pieces of the mass spectrometer (e.g., oven-heated column, autosampler injector) to specified temperature levels. In some instances, the instrument workflow can be indicated or provided by a user of the scientific instrument. For example, the user can interact with any suitable human-computer interface of the scientific instrument (e.g., touchscreen, keyboard, voice control system) so as to inform the scientific instrument of the instrument workflow. In any case, the scientific instrument can begin or attempt to perform the instrument workflow on the specimen.

In various aspects, the instrument workflow can be interrupted by an error message. That is, the scientific instrument can, while attempting to carry out whatever sequence of hardware or software operations make up the instrument workflow, suddenly or abruptly cease or pause the instrument workflow and generate the error message. In other words, the instrument workflow can be made up of a plurality of operations that are to be performed sequentially, and the scientific instrument can, after performing whatever first or initial operation is included in the instrument workflow but before performing whatever last or final operation is included in the instrument workflow, cease progressing through such plurality of operations and instead display the error message. In any case, the error message can be any suitable electronic data that indicates (explicitly or implicitly) that the scientific instrument is unable to complete or finish the instrument workflow due to experiencing some malfunction. As some examples, the error message can be: structured text (e.g., an alphanumeric identifier) that somehow corresponds to or is associated with the occurrence of that malfunction; unstructured text (e.g., one or more natural language sentences or sentence fragments) that semantically describes or states the occurrence or some other detail of the malfunction; or a visual symbol (e.g., a prohibited/slashed-circled symbol, a frowning-face symbol) that somehow visually indicates the occurrence or some other detail of the malfunction.

In various embodiments, the status component of the computerized tool can, in response to generation of the error message, electronically cause the scientific instrument to access or collect instrument runtime data. In various aspects, the instrument runtime data can be any suitable electronic data that the scientific instrument generated, logged, used, or otherwise tracked during its partial-performance or partial-run of the instrument workflow. As some examples, the runtime data can include: whatever values or states that the configurable operating parameters of the scientific instrument had during the partial-performance or partial-run of the instrument workflow; whatever imaging or spectrometry data that the scientific instrument partially captured during the partial-performance or partial-run of the instrument workflow; or measurements that were recorded, during the partial-performance or partial-run of the instrument workflow, by whatever auxiliary or ancillary sensors (e.g., pressure sensors, video cameras, microphones, temperature sensors, humidity sensors) that are incorporated or built into the scientific instrument. Accordingly, the runtime data can be considered as conveying the operational status of the scientific instrument leading up to and at the moment that the instrument workflow was interrupted by the malfunction.

In various embodiments, the model component of the computerized tool can electronically generate a solution to the malfunction, by executing the LLM on the error message and on the instrument runtime data.

More specifically, there can be a causation prompt, which can be unstructured or plain text that asks or commands that it be determined what caused the scientific instrument to generate the error message. In various aspects, the model component can concatenate the error message, the instrument runtime data, and the causation prompt together. In various instances, the model component can feed that concatenation to the input layer of the LLM, that concatenation can complete a forward pass through the one or more hidden layers of the LLM, and the output layer of the LLM can calculate a first natural language response based on activations provided by the one or more hidden layers of the LLM.

In various cases, the first natural language response can be synthesized text that is based on the error message and the instrument runtime data, and that substantively or semantically responds to the causation prompt. In other words, the first natural language response can be unstructured or plain text that describes or explains what (as inferred or predicted by the LLM) has caused the scientific instrument to interrupt the instrument workflow. In still other words, the first natural language response can textually state what hardware-related fault or software-related fault of the scientific instrument that the LLM believes was the root cause of the error message. That is, the error message and instrument runtime data can be considered as providing the LLM with a snap-shot of the inner workings of the scientific instrument during its attempt to perform the instrument workflow, and the LLM can utilize that snap-shot to infer or predict what specific piece of hardware or software of the scientific instrument failed and thereby interrupted the instrument workflow. Stated differently, the LLM can leverage any discrepancy, inconsistency, or other interrelationship between the inner workings that the scientific instrument would have been expected to have during performance of the instrument workflow and the inner workings that the scientific instrument actually had during the instrument workflow, so as to infer or predict why the instrument workflow was interrupted. For example, if the instrument workflow is a specific type of imaging protocol or imaging scan, then the LLM can expect the instrument runtime data to look or appear a certain way (e.g., to have or lack certain visual, audible, or numerical patterns or artifacts). How much or what ways the instrument runtime data diverges from such expectation can be considered as at least partially indicating or suggesting to the LLM what is wrong with the scientific instrument.

In some instances, there can be a resolution prompt, which can be unstructured or plain text that asks or commands that it be determined how to resolve or rectify whatever piece of hardware or software is specified in the first natural language response. In various aspects, the model component can concatenate the error message, the instrument runtime data, the first natural language response, and the resolution prompt together. In various instances, the model component can feed that concatenation to the input layer of the LLM, that concatenation can complete a forward pass through the one or more hidden layers of the LLM, and the output layer of the LLM can calculate a second natural language response based on activations provided by the one or more hidden layers of the LLM.

In various cases, the second natural language response can be synthesized text that is based on the error message, the instrument runtime data, and the first natural language response, and that substantively or semantically responds to the resolution prompt. In other words, the second natural language response can be unstructured or plain text that describes or explains what hardware adjustments or software adjustments (as inferred or predicted by the LLM) would cure whatever has caused the scientific instrument to interrupt the instrument workflow. In still other words, the second natural language response can textually state what sequence of maintenance, servicing, or development actions would, if conducted on the scientific instrument, cure or remedy whatever hardware or software faults are indicated in the first natural language response. That is, the error message and the instrument runtime data can be considered as providing the LLM with a snap-shot of the inner workings of the scientific instrument during its attempt to perform the instrument workflow, the first natural language response can be considered as indicating a specific hardware or software failure that has been inferred to have interrupted the instrument workflow, and the LLM can utilize such information to infer or predict what specific remedial steps should be taken to fix such hardware or software failure. After all, different types of instrument workflows can experience respective types of hardware or software failures as each other, and different types of hardware or software failures can require respective sequences of remedial steps.

In some instances, there can be a code prompt, which can be unstructured or plain text that asks or commands that a coding script be generated whose execution would implement whatever software actions or software steps are specified in the second natural language response. In various aspects, the model component can concatenate the error message, the instrument runtime data, the second natural language response, and the code prompt together. In various instances, the model component can feed that concatenation to the input layer of the LLM, that concatenation can complete a forward pass through the one or more hidden layers of the LLM, and the output layer of the LLM can compute a coding script based on activations provided by the one or more hidden layers of the LLM.

In various cases, the coding script can be written in any suitable programming syntax and can substantively or functionally satisfy the code prompt. In other words, the coding script can be synthesized lines of computer-executable code that are configured to, upon execution, perform or implement whatever remedial software actions or operations are specified in the second natural language response. In still other words, the second natural language response can semantically explain what software adjustments to make to a source code file of the scientific instrument so as to cure whatever malfunction interrupted the instrument workflow, and the coding script can be configured to actually implement or perform those software adjustments.

Now, in some cases, an accuracy level, completeness level, or amount of specificity or detail exhibited by the first natural language response, the second natural language response, or the coding script can be increased or otherwise improved by allowing the LLM to take into account supplemental information regarding the scientific instrument.

As a non-limiting example, there can be a document repository comprising a plurality of documents. In various instances, each of the plurality of documents can be any suitable electronic file (e.g., word-doc file, portable document format (PDF) file, webpage file) that can textually (or, in some cases, graphically or numerically) describe, explain, or otherwise indicate any suitable technical information regarding the design, fabrication, operation, maintenance, or troubleshooting of any suitable scientific instruments (e.g., various documents can be service manuals or technical handbooks (or portions thereof) of some respective scientific instruments). In various instances, any of the plurality of documents can be or have been written (e.g., via any suitable word processing software, computer-aided design software, or quantitative analysis software) by technicians or engineers who were tasked with designing, developing, prototyping, revising, manufacturing, or researching any suitable scientific instruments. Note that, in some cases, any document can exhibit or otherwise have any suitable length or size (e.g., can be one or a few pages in length; can be tens of pages in length; can be hundreds of pages in length). In any case, the model component can electronically search through the document repository for one or more documents that are substantively relevant to the error message or the instrument runtime data. In some aspects, the model component can accomplish this via an embedding search. For instance, the encoder portion of the LLM can be leveraged to generate a particular embedding for the error message and the instrument runtime data; the encoder portion can be leveraged to generate a respective embedding for each document in the document repository; and whichever documents whose embeddings are closest or most similar to the particular embedding can be considered as being relevant to the error message or the instrument runtime data. So, in various aspects, the model component can concatenate those relevant documents together with the error message and the instrument runtime data, and the model component can generate the first natural language response, the second natural language response, or the coding script by executing the LLM on that enlarged concatenation (in addition to any appropriate prompts). In various cases, the relevant documents can be considered as providing to the LLM deeper or richer information regarding how the scientific instrument is known or expected to perform various instrument workflows, how the scientific instrument is known or expected to respond to various types of malfunctions, how the scientific instrument should be repaired when afflicted with respective malfunctions, or how snippets of source code of the scientific instrument should be written or formatted, and such deeper or richer information can enable the LLM to make the first natural language response, the second natural language response, or the coding script more accurate or more detailed. It should be understood or otherwise appreciated that this can be considered as a utilization of RAG.

In any case, the first natural language response, the second natural language response, or the coding script can collectively be considered as the solution to whatever malfunction interrupted the instrument workflow.

In various embodiments, the solution generated by the LLM can be considered as being easy or not difficult to implement (e.g., changing only a small number software parameters, making a simple adjustment to a constituent piece of hardware). In such situations, the execution component of the computerized tool can electronically present the solution to a user of the scientific instrument in any suitable fashion. As a non-limiting example, the execution component can visually render the first or second natural language responses on any suitable computer screen or computer monitor that is associated with the scientific instrument, such that the user can view or read them. As another non-limiting example, the execution component can aurally play (e.g., via any suitable text-to-speech transformation techniques) the first or second natural language responses on any suitable speaker that is associated with the scientific instrument, such that the user can hear or listen to them. In this way, the computerized tool can be considered as telling or teaching the user what caused the instrument workflow to be interrupted and how the instrument workflow can be fixed. In cases where the solution includes the coding script, the execution component can electronically execute (or can electronically instruct the scientific instrument to execute) the coding script. Thus, whatever software adjustments that the LLM inferred would allow the instrument workflow to be completed can be actually implemented. After execution of the coding script, the workflow component can, in some cases, instruct the scientific instrument to retry or re-attempt the instrument workflow.

However, in other embodiments, the solution generated by the LLM can instead be considered as being difficult or not easy to implement (e.g., heavily editing or amending the source code of the scientific instrument, heavily redesigning the physical structure of the scientific instrument). In such situations, the execution component of the computerized tool can instead electronically present in any suitable fashion (e.g., via visual or audible rendition) the solution to a field engineer, technical specialist, or research scientist associated with a manufacturer of the scientific instrument. In such situations, the first natural language response and the second natural language response can be considered as eliminating the need for malfunction diagnosis or resolution brainstorming on the part of the field engineer, technical specialist, or research scientist. Additionally, in such situations, the coding script can be considered as reducing the amount of manual coding that the field engineer, technical specialist, or research scientist is required to do to resolve whatever malfunction is specified in the first natural language response. For example, rather than beginning to code from scratch, the field engineer, technical specialist, or research scientist can instead make whatever edits or changes they deem appropriate to the coding script. In any case, by providing the first natural language response, the second natural language response, and the coding script to the field engineer, technical specialist, or research scientist, the amount of time or effort that must be consumed by the field engineer, technical specialist, or research scientist in troubleshooting the scientific instrument (e.g., in developing or releasing a remedial patch or update for the scientific instrument) can be significantly reduced.

Now, in some embodiments, as mentioned above, one or more technical documents associated with the design, manufacture, operation, or servicing of the scientific instrument can be fed as RAG references to the LLM, so as to help the LLM more reliably or accurately synthesize the solution (e.g., the first natural language response, the second natural language response, or the coding script). In some aspects, a field engineer, technical specialist, or research scientist can provide to the computerized tool (e.g., via any suitable human-computer interface of the scientific instrument) feedback regarding the appropriateness or accuracy of the solution. In various instances, the feedback can be any suitable electronic data (e.g., structured or unstructured text) that identifies or specifies one or more troubleshooting inaccuracies or missteps that are present or contained in the solution. In various cases, the model component can utilize such feedback to help prevent the LLM from making those same troubleshooting inaccuracies or missteps in the future. Specifically, there can be an edit prompt, which can be unstructured or plain text that asks or commands that it be determined how whatever RAG references that the LLM relied upon to generate the solution be edited so as to avoid whatever inaccuracies or missteps are specified in the feedback. In various aspects, the model component can concatenate the solution, the RAG references, and the edit prompt together. In various instances, the model component can feed that concatenation to the input layer of the LLM, that concatenation can complete a forward pass through the one or more hidden layers of the LLM, and the output layer of the LLM can calculate one or more textual edits based on activations provided by the one or more hidden layers of the LLM.

In various cases, the one or more textual edits can be synthesized semantic markups that substantively satisfy the edit prompt. In other words, the one or more textual edits can be word deletions or word insertions which, if they had been applied to the RAG references before the LLM generated the solution, the solution would not have whatever troubleshooting inaccuracies or missteps are indicated in the feedback. In still other words, the feedback can be considered as specifying which portions of the solution were synthesized incorrectly, the LLM can be considered as predicting or inferring which pages, paragraphs, or sentences of the RAG references caused it to generate or create those the incorrect portions of the solution, and the LLM can be considered as further predicting or inferring how to linguistically edit or change those pages, paragraphs, or sentences of the RAG references so as to avoid making the same mistakes, inaccuracies, or missteps in the future. In some instances, this can be considered as an inverted-RAG technique (e.g., rather than utilizing references to synthesize text, this can be considered as utilizing feedback regarding synthesized text to change or edit references). In any case, the execution component can electronically implement or apply the one or more textual edits to whatever RAG references the LLM relied upon in synthesizing the solution. Thus, future solution synthesis performed by the LLM can have a higher likelihood of being more correct or more accurate (e.g., the one or more textual edits can be considered as fixing whatever parts of the RAG references that led the LLM astray, thereby making it less likely that the LLM will be led astray in the future).

In this way, the computerized tool can be considered as facilitating quick and effective automated troubleshooting in response to interruption of scientific instrument workflows. Thus, the amount of time that users of scientific instruments must wait for hardware or software modifications to be provided in response to scientific instrument malfunctions, as well as the amount of effort that must be expended by instrument manufacturers to brainstorm and develop such hardware or software modifications, can be significantly reduced.

Various embodiments described herein can be employed to use hardware or software to solve problems that are highly technical in nature (e.g., to facilitate large language model resolution of scientific instrument workflow interruptions), that are not abstract and that cannot be performed as a set of mental acts by a human. Further, some of the processes performed can be performed by a specialized computer (e.g., electron microscopes such as SEMs, TEMs, or EELMs; mass spectrometers coupled to liquid, gas, or ion chromatographs; artificial neural networks such as LLMs) for carrying out defined acts related to the field of scientific instruments.

For example, such defined acts can include: causing, by a device operatively coupled to a processor, a scientific instrument (e.g., charged-particle microscope, mass spectrometer) to perform a workflow on a specimen; retrieving, by the device and in response to the scientific instrument generating an error message that interrupts the workflow, runtime data logged by the scientific instrument during the workflow; and synthesizing, by the device and via execution of a large language model on the error message and on the runtime data, first text that explains why the workflow was interrupted. Such defined acts can further include: synthesizing, by the device and via execution of the large language model on the first text, second text that explains how to resolve the workflow. In various aspects, such defined acts can further include: synthesizing, by the device and via execution of the large language model on the second text, a coding script that is configured to cause the scientific instrument to resolve the workflow; and causing, by the device, the scientific instrument to execute the coding script, thereby resolving the workflow. In various cases, the workflow can be: an imaging scan of the specimen; a milling operation of the specimen; a movement of an actuatable stage holding the specimen; a spectrometry scan of the specimen; or an injection or flushing operation of the specimen. In various aspects, the runtime data can include: a partial charged-particle image or partial mass spectrum of the specimen captured by the scientific instrument during the workflow; or states that one or more configurable operating parameters of the scientific instrument had during the workflow. In various instances, the large language model can synthesize the first text in retrieval-augmented generative fashion based on performing an embedding search through a repository of technical documentation associated with a design, manufacture, operation, or troubleshooting of the scientific instrument, and the defined tasks can further include: receiving, by the device, feedback from a user or technician regarding the first text; synthesizing, by the device and via execution of the large language model on the feedback, a textual edit to a document in the repository of technical documentation that was retrieved by the embedding search; and updating, by the device, the repository of technical documentation by inserting the textual edit into the document.

Such defined acts are inherently computerized. Indeed, a scientific instrument, such as a charged-particle microscope (e.g., SEM, TEM, EELM, dual beam microscope) or a mass spectrometer coupled to a chromatograph (e.g., liquid chromatograph, gas chromatograph, ion chromatograph), is a highly-technical computerized device comprising specific computerized hardware (e.g., temperature sensors, pressure sensors, voltage sensors, ion beam emitters, electron beam emitters, focusing lenses, ion detectors, electron detectors, beam apertures, fluid valves, actuatable specimen stages). A scientific instrument, the operations that it performs, and the electronic data that it captures cannot be implemented by the human mind, or by a human with pen and paper, in any reasonable or practicable way without computers. Furthermore, artificial neural networks (e.g., LLMs) are also inherently computerized constructs comprising specific software-oriented architectures (e.g., input layers, hidden layers, or output layers, any of which can be made up of trainable or non-trainable internal parameters such as convolutional layers or LSTM layers). Artificial neural networks cannot be trained or executed by the human mind, or by humans with mere pen and paper, in any reasonable or practicable way without computers.

Moreover, various embodiments described herein can integrate into a practical application various teachings relating to the field of scientific instruments. As explained above, scientific instruments are highly complex, complicated devices that often require extensive specialized training, education, or certification to learn how to operate, maintain, or troubleshoot. Accordingly, when a scientific instrument encounters a malfunction, a user can be unable to confidently troubleshoot that malfunction themself and can instead be forced to wait for troubleshooting to be performed by a technical service engineer employed by a manufacturer of the scientific instrument. Unfortunately, it can take hours or days before any technical service engineer becomes available to even consider the malfunction. Moreover, once the technical service engineer finally begins to consider the malfunction, the amount of time required for the technical service engineer to diagnose and solve that malfunction can range from as little as a few minutes to as long as several weeks or months, depending upon the complexity of the malfunction. Indeed, low-complexity malfunctions might be easily solved by merely changing the values of a small number of configurable operating parameters of the scientific instrument, which can take mere minutes; intermediate-complexity malfunctions might require more extensive editing to be performed on the source code of the scientific instrument, which can take days; and high-complexity malfunctions might require entire new pages of source code to be crafted from scratch (or might even require hardware of the scientific instrument to be redesigned), which can take weeks or months.

Various embodiments described herein can help to ameliorate this problem, by implementing large language model resolution of scientific instrument workflow interruptions. That is, various embodiments described herein can leverage LLMs so as to reduce an amount of time or effort expended in troubleshooting scientific instrument malfunctions. As described herein, various embodiments can accomplish such goal by: interruption-triggered LLM resolution synthesis; or feedback-conditioned LLM editing of RAG references.

Indeed, when performing a workflow (e.g., an imaging scan, a spectrometry scan, a specimen repositioning operation, a specimen heating or cooling operation), a scientific instrument can be suddenly or abruptly interrupted by an error message, thereby leaving the workflow uncompleted. Various embodiments described herein can, in response to such interruption, collect whatever runtime data (e.g., partial or unfinished images, partial or unfinished spectra, currently-assigned operating parameter values, auxiliary sensor feeds) the scientific instrument generated or utilized during the partial-performance of the workflow and can feed both the error message and the runtime data as inputs to the LLM. As described herein, when such inputs are accompanied by appropriate prompts (e.g., the herein-described causation prompt, the herein-described resolution prompt, the herein-described coding prompt), this can cause the LLM to synthesize textual content that serves to troubleshoot the error message. In some cases, that synthesized textual content can explain what seems to have caused the error message. In other cases, that synthesized textual content can explain how to rectify or eliminate the underlying cause of the error message. In even other cases, that synthesized textual content can include executable lines of computer code which are configured to rectify or eliminate the underlying cause of the error message. In any case, such synthesized textual content can be created in mere seconds after the occurrence of the error message. Contrast this with the hours, days, weeks, or even months that field engineers or technical service specialists could potentially spend evaluating, brainstorming, and developing a solution, repair, or patch to the error message. Additionally, as described herein, the LLM can generate such synthesized textual content by relying upon one or more RAG references that contain technical information about the scientific instrument. Sometimes, RAG references can mistakenly become outdated. In some aspects, various embodiments can involve leveraging the LLM to update or edit RAG references, in response to feedback that indicates that the synthesized textual content was somehow less than satisfactory. This concept can be considered as a type of inverted-RAG and can help to prevent the LLM from repeating any troubleshooting mistakes that the feedback identifies.

Furthermore, it must be emphasized how clever and counterintuitive various embodiments described herein are. Indeed, various embodiments described herein can be considered as conditioning LLM synthesis of troubleshooting advice or solutions on runtime data that a scientific instrument collects during an interrupted or incomplete workflow. Such runtime data can, in some instances, include unfinished or partially-captured electron images, unfinished or partially-captured chromatograms, or unfinished or partially-captured mass spectra. Conventionally, such unfinished or partially-captured data is considered to be corrupted, meaningless, and otherwise useless. Accordingly, conventional techniques discard such unfinished or partially-captured data. In stark contrast, the inventors of various embodiments described herein realized that such unfinished or partially-captured data can, notwithstanding being incomplete, nevertheless contain potentially valuable clues as to what caused the scientific instrument's workflow to be interrupted. In other words, the present inventors recognized that whatever specific type of malfunction that the scientific instrument experienced can leave hidden traces, signatures, or patterns in such unfinished or partially-captured data and that the LLM can utilize such hidden traces, signatures, or patterns to generate troubleshooting inferences for the scientific instrument (e.g., different types of malfunctions, and thus different types of malfunction resolutions, can cause the unfinished or partially-captured data to look or appear in respective ways). Because conventional wisdom teaches that such unfinished or partially-captured data is useless and should be discarded, various embodiments described herein that utilize such unfinished or partially-captured data so as to reduce the amount of time or effort required for troubleshooting can be considered as being highly counterintuitive, unexpected, unusual, or clever.

For at least the above reasons, various embodiments described herein can be considered as addressing or ameliorating various problems or disadvantages regarding troubleshooting of scientific instruments. Therefore, various embodiments described herein can be considered as a concrete and tangible technical improvement in the field of scientific instruments. Accordingly, various embodiments described herein certainly qualify as useful and practical applications of computers.

Furthermore, various embodiments described herein can control real-world tangible devices based on the disclosed teachings. For example, various embodiments described herein can electronically activate, deactivate, or otherwise actuate real-world hardware (e.g., ion beam emitters, ion focusing lenses, carrier fluid valves/pumps) of real-world scientific instruments (e.g., SEMs, TEMs, EELMs, dual-beam microscopes, mass spectrometers, gas chromatographs).

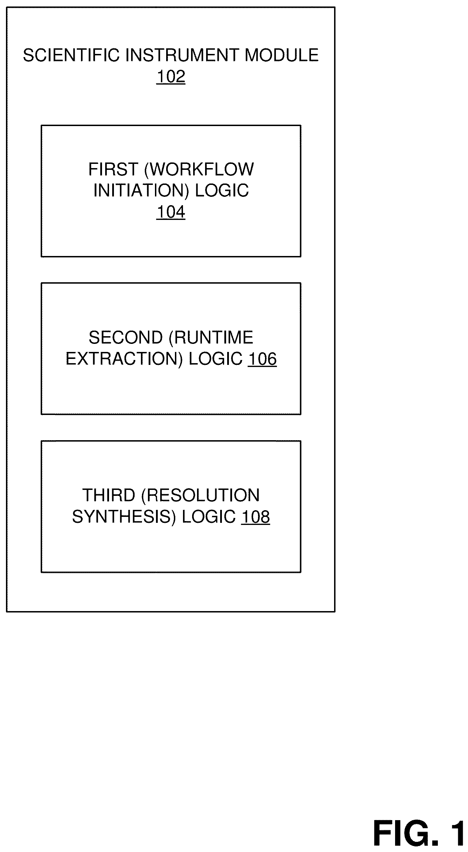

FIG. 1 illustrates an example, non-limiting block diagram of a scientific instrument module 102 in accordance with various embodiments described herein.

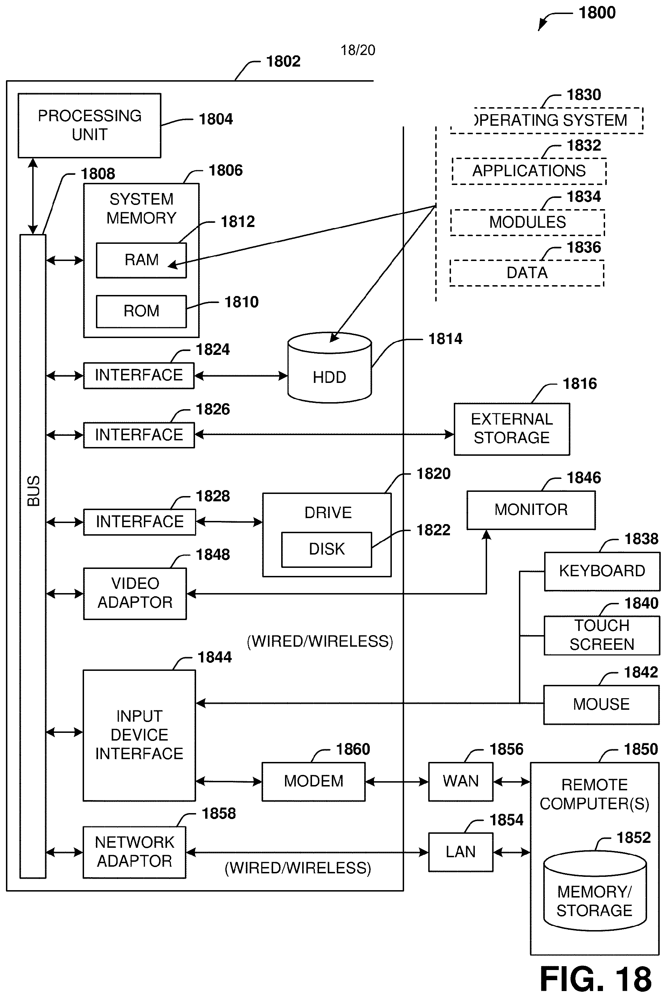

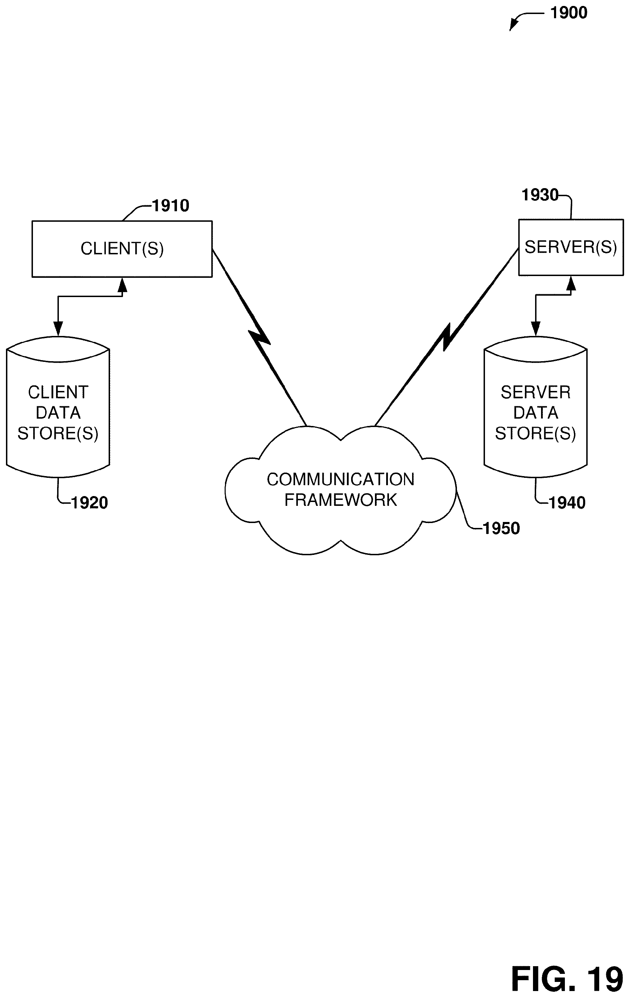

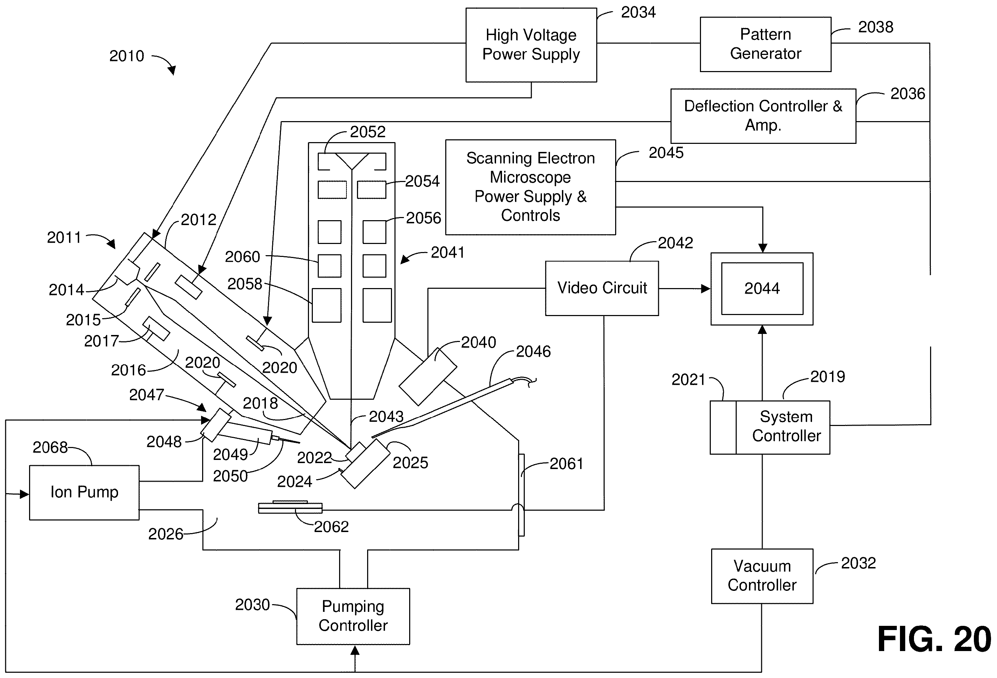

In various embodiments, the scientific instrument module 102 can be implemented by circuitry (e.g., including electrical or optical components), such as a programmed computing device. Logic of the scientific instrument module 102 can be included in a single computing device or can be distributed across multiple computing devices that are in communication with each other as appropriate. Examples of computing devices that may, singly or in combination, implement the scientific instrument module 102 are discussed herein with reference to FIG. 18 and 20, and examples of systems or networks of interconnected computing devices, in which the scientific instrument module 102 may be implemented across one or more of the computing devices, are discussed herein with reference to FIG. 19.

The scientific instrument module 102 can include first logic 104, second logic 106, and third logic 108. As used herein, the term "logic" can include an apparatus that is to perform a set of operations associated with the logic. For example, any of the logic elements included in the scientific instrument module 102 can be implemented by one or more computing devices programmed with instructions to cause one or more processing devices of the computing devices to perform the associated set of operations. In a particular embodiment, a logic element may include one or more non-transitory computer-readable media having instructions thereon that, when executed by one or more processing devices of one or more computing devices, cause the one or more computing devices to perform the associated set of operations. As used herein, the term "module" can refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module can omit one or more of the logic elements depicted in the associated drawings; for example, a module may include a subset of the logic elements depicted in the associated drawings when that module is to perform a subset of the operations discussed herein with reference to that module.

In various embodiments, there can be a scientific instrument corresponding to the scientific instrument module 102. In various aspects, the scientific instrument can be any suitable computerized device that can electronically measure some scientifically-relevant, clinically-relevant, or research-relevant characteristic, property, or attribute of an analytical specimen (e.g., of a known or unknown mixture, compound, or collection of matter). As a non-limiting example, a scientific instrument can be a scanning electron microscope. In such case, the scientific instrument can measure or determine a surface topography of the analytical specimen. As another non-limiting example, a scientific instrument can be a transmission electron microscope. In such case, the scientific instrument can measure or determine internal structural details of the analytical specimen. As yet another non-limiting example, a scientific instrument can be an electron energy-loss microscope. In such case, the scientific instrument can measure or determine location-wise counts or intensities across a range of defined energy-loss bins or bands for the analytical specimen. As a more general non-limiting example, a scientific instrument can be any suitable type of charged-particle microscope (e.g., some types of microscopes can use beams of non-electron ions to capture images or energy spectra or to otherwise interact with specimens). As another non-limiting example, a scientific instrument can be a mass spectrometer that is operatively coupled to a chromatograph. In such case, the scientific instrument can measure or determine chromatograms (e.g., relative compound abundance as a function of retention time) or ion spectra (e.g., relative ion abundance as a function of mass-to-charge ratio) of the analytical sample.

In various embodiments, the first logic 104 can cause the scientific instrument to perform a workflow (e.g., imaging scan, spectrometry screening, milling, heating, cooling, repositioning) on a specimen.

In various embodiments, the second logic 106 can involve retrieving, in response to the scientific instrument generating an error message that interrupts the workflow, runtime data (e.g., partially-captured images, partially-captured spectra, operating parameter values) logged by the charged-particle microscope during the workflow.

In various embodiments, the third logic 108 can involve synthesizing, via execution of a large language model on the error message and on the runtime data, first text that explains why the workflow was interrupted. In various aspects, the third logic 108 can further include synthesizing, via execution of the large language model on the first text, second text that explains how to resolve the workflow. In various instances, the third logic 108 can further include synthesizing, by the device and via execution of the large language model on the second text, a coding script that is configured to cause the scientific instrument to resolve the workflow. In some cases, the large language model can synthesize the first text, second text, or coding script in retrieval-augmented generative fashion based on performing an embedding search through a repository of technical documentation associated with a design, manufacture, operation, or troubleshooting of the scientific instrument. In such situations, the third logic 108 can further include: receiving feedback from a user or technician regarding the first text; second text, or coding script; synthesizing, via execution of the large language model on the feedback, a textual edit to a document in the repository of technical documentation that was retrieved by the embedding search; and updating the repository of technical documentation by inserting the textual edit into the document.

Accordingly, the scientific instrument module 102 can facilitate large language model resolution of scientific instrument workflow interruptions.

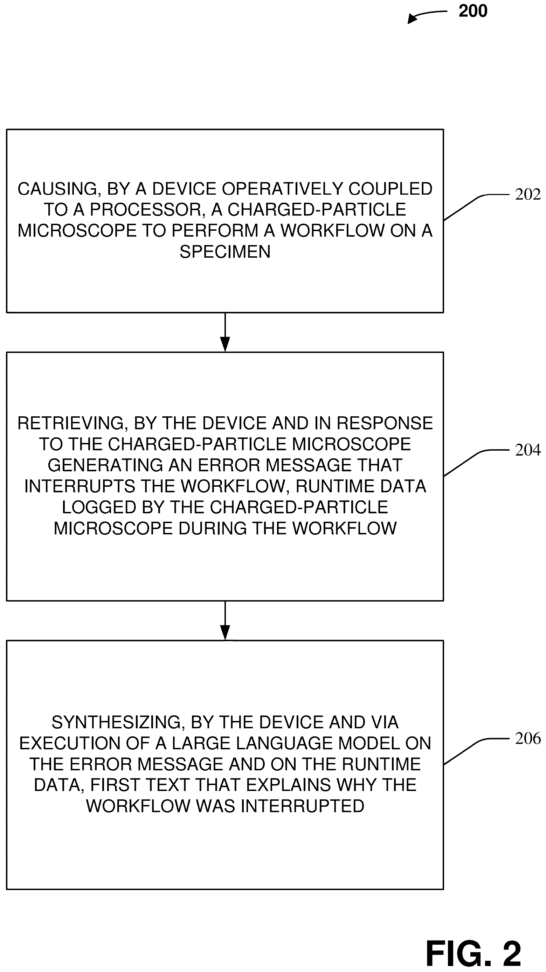

FIG. 2 is an example, non-limiting flow diagram of a computer-implemented method 200 in accordance with various embodiments described herein. The operations of the computer-implemented method 200 may be used in any suitable context to perform any suitable operations (e.g., can be performed by or used in conjunction with any of the various modules, computing devices, or graphical user interfaces described with respect to of FIGs. 1, 18, 19, and 20). Operations are illustrated once each and in a particular order in FIG. 2, but the operations may be reordered or repeated as desired and appropriate (e.g., different operations performed may be performed in parallel, as suitable).

In various aspects, act 202 can include performing first operations causing, by a device operatively coupled to a processor, a scientific instrument (e.g., charged-particle microscope) to perform a workflow on a specimen. In various cases, the first logic 104 can perform or otherwise facilitate act 202.

In various aspects, act 204 can include performing second operations retrieving, by the device and in response to the scientific instrument generating an error message that interrupts the workflow, runtime data logged by the scientific instrument during the workflow. In various instances, the second logic 106 can perform or otherwise facilitate act 204.

In various cases, act 206 can include performing third operations synthesizing, by the device and via execution of a large language model on the error message and on the status data, first text that explains why the workflow was interrupted.

Accordingly, the computer-implemented method 200 can facilitate large language model resolution of scientific instrument workflow interruptions.

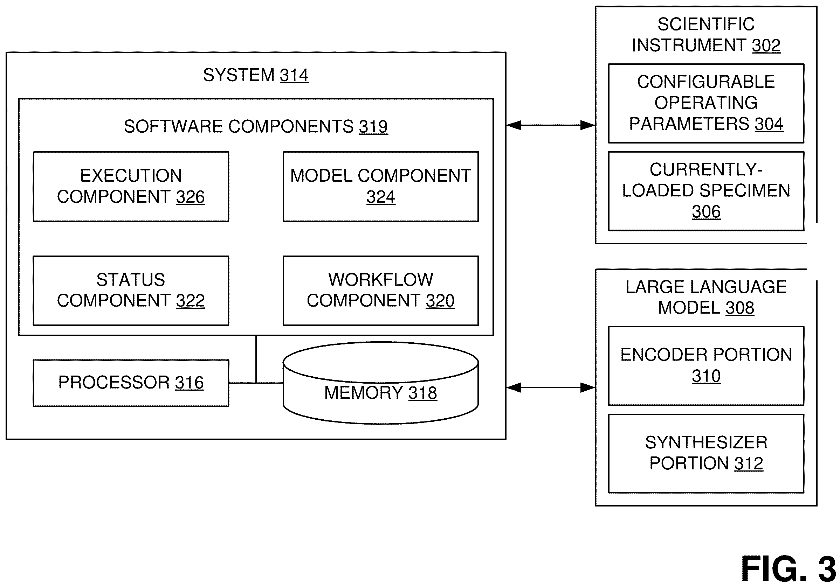

FIG. 3 illustrates a block diagram of an example, non-limiting system that facilitates large language model resolution of scientific instrument workflow interruptions in accordance with one or more embodiments described herein.

In various embodiments, there can be a scientific instrument 302. In various aspects, the scientific instrument 302 can be as described above. That is, the scientific instrument 302 can be any suitable computerized that device that can leverage its constituent hardware (e.g., electron sources, anodes, condenser lenses, condenser apertures, scan coils, objective lenses, objective apertures, deflectors, condensers, stigmators, electron detectors, X-ray detectors, actuatable specimen stages, autosampler injectors, oven-heated columns, mass analyzers, absorbent packing materials, stationary phase films, vacuum chambers, fluid conduits) to electronically capture any suitable type of measurable or quantifiable data of any suitable analytical specimen. As a non-limiting example, the scientific instrument 302 can be any suitable type of charged-particle microscope (e.g., an SEM; a TEM; an EELM; a dual-beam microscope) that can electronically capture or generate any suitable types of charged-particle images of a surface portion or interior portion of an analytical specimen. As another non-limiting example, the scientific instrument 302 can be any suitable chromatograph (e.g., liquid chromatograph; gas chromatograph; ion chromatograph; thin-layer chromatograph; affinity chromatograph) or mass spectrometer (e.g., quadrupole mass spectrometer; time-of-flight mass spectrometer; ion trap mass spectrometer; Fourier transform ion cyclotron resonance mass spectrometer; magnetic sector mass spectrometer) that can electronically capture any suitable types of compositional ion abundance data (e.g., chromatograms, mass spectra) associated with an analytical specimen.

Although not explicitly shown in the figures, the scientific instrument 302 can be electronically integrated with any suitable human-computer interface device, which can be remote from or local to the scientific instrument 302. Accordingly, a user or technician associated with the scientific instrument 302 can interact with or otherwise control the scientific instrument 302. Some non-limiting examples of the human-computer interface device can be a keyboard of the scientific instrument 302, a keypad of the scientific instrument 302, a touchscreen of the scientific instrument 302, or a voice-command system of the scientific instrument 302.

In any case, the scientific instrument 302 can comprise a plurality of configurable operating parameters 304. In various aspects, each of the plurality of configurable operating parameters 304 can be any suitable hardware-related characteristic or software-related characteristic of the scientific instrument 302 that can guide, affect, or otherwise dictate how the scientific instrument 302 runs, operates, or functions with respect to any given analytical specimen and that can be selectively controlled, changed, adjusted, or otherwise set by the user or technician (e.g., via interaction with the human-computer interface device of the scientific instrument 302). As a non-limiting example, any of the plurality of configurable operating parameters 304 can be a user-controllable electric voltage setting (e.g., beam voltage) or electric current setting (e.g., beam current), which can allow the user or technician to selectively control an electrode of the scientific instrument 302, so as to selectively increase or decrease an electric voltage or electric current within, or that is applied by, the scientific instrument 302. As another non-limiting example, any of the plurality of configurable operating parameters 304 can be a user-controllable temperature setting, which can allow the user or technician to control a heater (e.g., stage heater, heating coil) or cooler (e.g., cooling fan, heat pump, refrigerator) of the scientific instrument 302, so as to selectively increase or decrease a temperature within, or that is applied by, the scientific instrument 302. As still another non-limiting example, any of the plurality of configurable operating parameters 304 can be a user-controllable mechanical actuator setting, which can allow the user or technician to control a mechanical actuator (e.g., electric motor, specimen stage, iris aperture, fluid pump or syringe, specimen-handling robotic arm) of the scientific instrument 302, so as to selectively move the mechanical actuator. As yet another non-limiting example, any of the plurality of configurable operating parameters 304 can be a user-controllable optics setting, which can allow the user or technician to control an optical element (e.g., optical lens, optical deflector) of the scientific instrument 302, so as to selectively change an optical quality (e.g., focal spot size or location, astigmatism, defocus) that is applied by the scientific instrument 302.

In various aspects, the scientific instrument 302 can have or otherwise be associated with a currently-loaded specimen 306. In various instances, the currently-loaded specimen 306 can (as its name suggests) be presently loaded on or within the scientific instrument 302. As a non-limiting example, in situations where the scientific instrument 302 is a charged-particle microscope, the currently-loaded specimen 306 can be presently positioned, located, or otherwise affixed onto an actuatable stage of the scientific instrument 302, such that the currently-loaded specimen 306 is analyzable or scannable by the scientific instrument 302. As another non-limiting example, in situations where the scientific instrument 302 is a chromatograph or mass spectrometer, the currently-loaded specimen 306 can be presently positioned, located, or otherwise contained within an autosampler injector of the scientific instrument 302, such that the currently-loaded specimen 306 is analyzable or scannable by the scientific instrument 302. In various cases, the currently-loaded specimen 306 can be any suitable type of medical, clinical, scientific, or laboratory sample that can exhibit any suitable physical, chemical, compositional, or other properties, attributes, or characteristics. As a non-limiting example, the currently-loaded specimen 306 can be a lamella taken from a semiconductor substrate or wafer. As another non-limiting example, the currently-loaded specimen 306 can be a sample or fragment taken from a failed load-bearing structure. As even another non-limiting example, the currently-loaded specimen 306 can be an aqueous solution or mixture containing any suitable type of solvents or solutes.

In various embodiments, there can be a large language model 308 (hereafter "LLM 308"). In various aspects, the LLM 308 can comprise an encoder portion 310 and a synthesizer portion 312. In various cases, the encoder portion 310 can be considered as being upstream from the synthesizer portion 312. Equivalently, the synthesizer portion 312 can be considered as being downstream of the encoder portion 310.

In various aspects, the encoder portion 310 can exhibit any suitable deep learning internal architecture. Indeed, in various cases, the encoder portion 310 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. As even another example, any of such input layer, one or more hidden layers, or output layer can be LSTM layers, whose learnable or trainable parameters can be input-state weight matrices or hidden-state weight matrices. As yet another example, any of such input layer, one or more hidden layers, or output layer can be transformer layers, whose learnable or trainable parameters can be single-head or multi-head attention blocks or other weight matrices. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

Likewise, in various instances, the synthesizer portion 312 can exhibit any suitable deep learning internal architecture. Indeed, in various cases, the synthesizer portion 312 can have an input layer, one or more hidden layers, and an output layer. In various instances, any of such layers can be coupled together by any suitable interneuron connections or interlayer connections (e.g., forward connections, skip connections, recurrent connections). Furthermore, in various cases, any of such layers can be any suitable types of neural network layers having any suitable learnable or trainable internal parameters (e.g., any of such input layer, one or more hidden layers, or output layer can be convolutional layers, dense layers, batch normalization layers, LSTM layers, or transformer layers). Further still, in various cases, any of such layers can be any suitable types of neural network layers having any suitable fixed or non-trainable internal parameters (e.g., any of such input layer, one or more hidden layers, or output layer can be non-linearity layers, padding layers, pooling layers, or concatenation layers).

Regardless of the specific internal architecture (e.g., the specific numbers, types, or organizations of layers) that is implemented within the encoder portion 310, the encoder portion 310 can be configured to receive textual data (which can be accompanied by any suitable numerical or graphical data) and to produce embeddings based on such inputted textual data. In contrast, regardless of the specific internal architecture that is implemented within the synthesizer portion 312, the synthesizer portion 312 can be configured to receive embeddings produced by the encoder portion 310 and to produce synthesized textual content based on such embeddings. As some non-limiting examples, the LLM 308 can be any of the following: ChatGPT; Gene.Al; Ollama; Bard; or Claude.

In various embodiments, a system 314 can be electronically integrated (e.g., via any suitable wired or wireless electronic connections) with the scientific instrument 302 or with the LLM 308. As described herein, the system 314 can leverage the LLM 308 so as to expedite troubleshooting of the scientific instrument 302 (e.g., reducing days, weeks, or months of troubleshooting or solution development to mere seconds or minutes).

In various aspects, the system 314 can comprise a processor 316 (e.g., computer processing unit, microprocessor) and a non-transitory computer-readable memory 318 that is operably or operatively or communicatively connected or coupled to the processor 316. The non-transitory computer-readable memory 318 can store computer-executable instructions which, upon execution by the processor 316, can cause the processor 316 or other components of the system 314 (e.g., workflow component 320, status component 322, model component 324, execution component 326) to perform one or more acts. In various embodiments, the non-transitory computer-readable memory 318 can store computer-executable components (e.g., workflow component 320, status component 322, model component 324, execution component 326), and the processor 316 can execute the computer-executable components.

In various embodiments, the system 314 can electronically access the scientific instrument 302 or the LLM 308. That is, the system 314 can electronically communicate or otherwise electronically interact with (e.g., transmit electronic instructions or commands to, receive electronic data from) the scientific instrument 302 or the LLM 308 in any suitable fashion. Accordingly, any suitable components of the system 314 can interact with, communicate with, or otherwise manipulate the scientific instrument 302 or the LLM 308. Note that the system 314 can, in some cases, be implemented on or hosted by the scientific instrument 302 itself or any suitable computerized workstation that is associated with or coupled to the scientific instrument 302. In such situations, the system 314 can be considered as being deployed in a client-side fashion (e.g., the system 314 can be considered as being local to the scientific instrument 302). However, in other cases, the system 314 can instead be implemented or hosted remotely from the scientific instrument 302, such as in a cloud computing environment. In such situations, the system 314 can be considered as being deployed in a server-side fashion.

In various embodiments, the system 314 can include a workflow component 320. In various aspects, the workflow component 320 can, as described herein, cause the scientific instrument 302 to initiate an instrument workflow.

In various embodiments, the system 314 can include a status component 322. In various instances, the status component 322 can, as described herein, collect runtime data generated or utilized by the scientific instrument 302, in response to the instrument workflow being interrupted by an error message.

In various embodiments, the system 314 can include a model component 324. In various cases, the model component 324 can, as described herein, leverage the runtime data and the LLM 308 so as to troubleshoot the error message that interrupts the instrument workflow.

In various embodiments, the system 314 can include an execution component 326. In various aspects, the execution component 326 can, as described herein, render, share, or otherwise implement whatever troubleshooting results are generated by the LLM.

Note that, in various instances, the workflow component 320, the status component 322, the model component 324, and the execution component 326 can collectively be considered as being one or more software components 319 of the system 314. In various aspects, it should be appreciated that the one or more software components 319 are described primarily herein as comprising four components (e.g., the workflow component 320, the status component 322, the model component 324, and the execution component 326) for ease of explanation and illustration. However, the one or more software components 319 are not limited to being implemented as exactly such four components in every embodiment. Indeed, in some embodiments, the functionalities described herein of such four components can be combined in any suitable fashions, so as to be implemented in or by fewer than four components (e.g., in some cases, a single component can perform all of the functionalities that are described herein with respect to the workflow component 320, the status component 322, the model component 324, and the execution component 326). In other embodiments, the functionalities described herein of such four components can instead be distributed, separated, split, or fragmented in any suitable fashions, so as to be implemented in or by more than four components (e.g., two or more components can facilitate the functionalities that are performable by the workflow component 320; two or more components can facilitate the functionalities that are performable by the status component 322; two or more components can facilitate the functionalities that are performable by the model component 324; two or more components can facilitate the functionalities that are performable by the execution component 326).

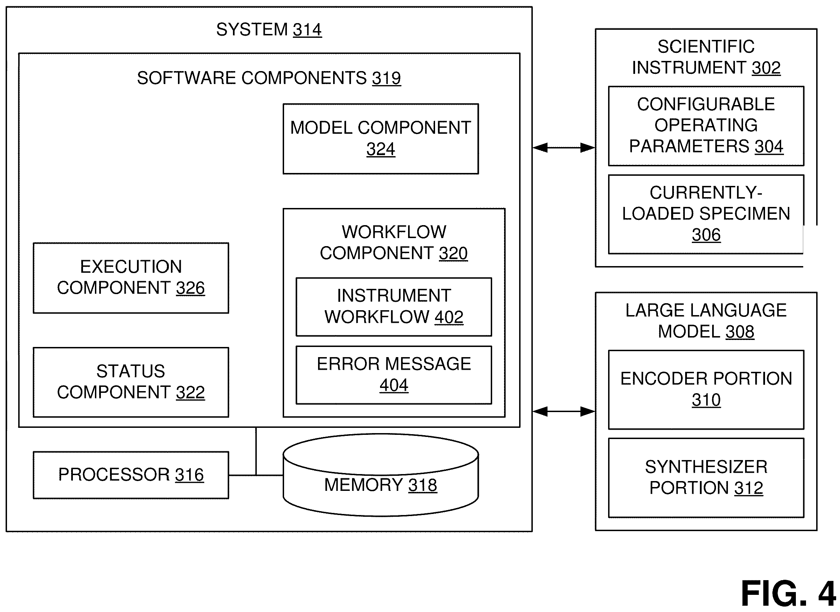

FIG. 4 illustrates a block diagram of an example, non-limiting system including an instrument workflow and an error message that can facilitate large language model resolution of scientific instrument workflow interruptions in accordance with one or more embodiments described herein.

In various embodiments, the workflow component 320 can electronically command, electronically instruct, or otherwise electronically cause the scientific instrument 302 to begin, initiate, or otherwise commence performance of an instrument workflow 402. In various aspects, the instrument workflow 402 can be any suitable sequence of one or more hardware operations or software operations that are performable, conductible, or doable by the scientific instrument 302. As a non-limiting example, in situations where the scientific instrument 302 is a charged-particle microscope, the instrument workflow 402 can be a charged-particle imaging protocol. In such case, the instrument workflow 402 can be considered as specifying whatever sequence of operations or actions (e.g., establishing desired focal spot size, establishing desired beam voltage or current, establishing desired beam raster pattern) that would generate a desired charged-particle image (e.g., pixel array, voxel array) of the surface of the currently-loaded specimen 306 or of an interior volume of the currently-loaded specimen 306. As another non-limiting example, in situations where the scientific instrument 302 is a charged-particle microscope, the instrument workflow 402 can be a milling protocol. In such case, the instrument workflow 402 can be considered as specifying whatever sequence of operations or actions (e.g., decreasing focal spot size, increasing beam voltage or current, aiming beam at a desired location on the currently-loaded specimen 306, injecting reactive gases onto the currently-loaded specimen 306) that would remove or etch a desired amount of material away from (or that could possibly deposit a desired amount of material onto) the currently-loaded specimen 306. As yet another non-limiting example, in situations where the scientific instrument 302 is a charged-particle microscope, the instrument workflow 402 can be a spatial repositioning protocol. **In** such case, the instrument workflow 402 can be considered as specifying whatever sequence of operations or actions (e.g., establishing desired angular or translational positions of an actuatable stage of the scientific instrument 302, causing a robotic arm of the scientific instrument 302 to extend, retract, or otherwise articulate in a desired fashion) that move or reorient the currently-loaded specimen 306 in any desired fashion. As still another non-limiting example, in situations where the scientific instrument 302 is a charged-particle microscope, the instrument workflow 402 can be a vacuum protocol. In such case, the instrument workflow 402 can be considered as specifying whatever sequence of operations or actions (e.g., opening or closing a load-lock door of the scientific instrument 302, activating or deactivating a vacuum pump of the scientific instrument 302) that cause a vacuum chamber of the scientific instrument 302 to achieve any desired pressure. As even another non-limiting example, in situations where the scientific instrument 302 is a chromatograph or mass spectrometer, the instrument workflow 402 can be a scanning or screening protocol. In such case, the instrument workflow 402 can be considered as specifying whatever sequence of operations or actions (e.g., heating a column oven of the scientific instrument 302 to a desired temperature, activating injection pumps or syringes of the scientific instrument 302, establishing a desired ion monitoring range of the scientific instrument 302) that cause the scientific instrument 302 to scan or screen the currently-loaded specimen 306 for any desired chemical compounds or molecules. As another non-limiting example, in situations where the scientific instrument 302 is a chromatograph or mass spectrometer, the instrument workflow 402 can be a specimen heating protocol. **In** such case, the instrument workflow 402 can be considered as specifying whatever sequence of operations or actions (e.g., heating an autosampler of the scientific instrument 302) that cause the scientific instrument 302 to bring the currently-loaded specimen 306 to a desired temperature. As still another non-limiting example, in situations where the scientific instrument 302 is a chromatograph or mass spectrometer, the instrument workflow 402 can be a specimen flushing protocol. In such case, the currently-loaded specimen 306 might be a cleaning agent rather than a research curiosity, and so the instrument workflow 402 can be considered as specifying whatever sequence of operations or actions (e.g., establishing desired fluid flow rates or fluid pressures of an autosampler injector of the scientific instrument 302) that cause the scientific instrument 302 to flush or cleanse its internal conduits or pipes with the currently-loaded specimen 306 in a desired fashion.

Thus, the instrument workflow 402 can be any suitable series or chain of automated hardware actions or automated software actions that can be performed by the scientific instrument 302, so as to achieve some desired result or effect with respect to the currently-loaded specimen 306. However, these are mere non-limiting instances. In some aspects, the currently-loaded specimen 306 can be omitted. That is, the scientific instrument 302 might be empty or might otherwise not have any specimen loaded inside of it but it can nevertheless be desired for the scientific instrument 302 to perform the instrument workflow 402 (e.g., there can be some workflows that are desired or intended to be performed without specimens).

In various instances, the instrument workflow 402 can be chosen or specified by a user or technician associated with the scientific instrument 302. As a non-limiting example, the user or technician can interact with or otherwise use the human-computer interface of the scientific instrument 302, so as to select the instrument workflow 402 from a defined list of available instrument workflows. As another non-limiting example, the user or technician can interact with or otherwise use the human-computer interface of the scientific instrument 302, so as to define in customized or ad hoc fashion the instrument workflow 402.

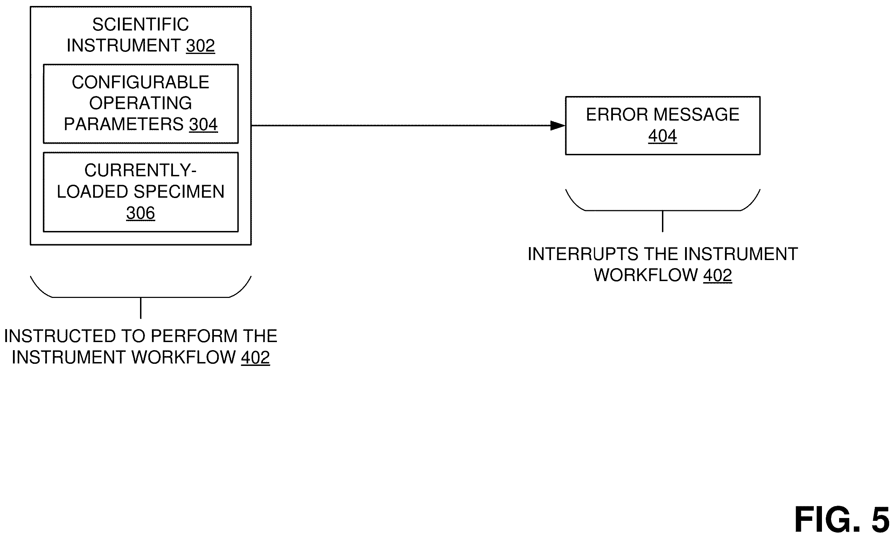

In any case, the workflow component 320 can cause the scientific instrument 302 to begin performing whatever sequence of actions makes up the instrument workflow 402. In various aspects, the scientific instrument 302 can, while performing the instrument workflow 402, be abruptly, suddenly, unexpectedly, or otherwise undesirably interrupted by the error message 404. Non-limiting details are described with respect to FIG. 5.

FIG. 5 illustrates an example, non-limiting block diagram showing how the error message 404 can interrupt the instrument workflow 402 in accordance with one or more embodiments described herein.

Suppose that the instrument workflow 402 is a sequence of x operations for any suitable positive integer x > 1, where each of such x operations can be performed or conducted by the scientific instrument 302. In response to a command or instruction from the workflow component 320, the scientific instrument 302 can begin progressing its way through that sequence of x operations, beginning or starting with an initial operation in the sequence and ending or finishing with an x-th operation in the sequence. During performance of the instrument workflow 402, the scientific instrument 302 can experience a malfunction. Such malfunction can cause or force the scientific instrument 302 to pause the instrument workflow 402 and to instead electronically generate, produce, or display the error message 404. For example, suppose that the malfunction occurs when the scientific instrument 302 is attempting to perform a j-th operation of the instrument workflow 402, for any suitable positive integer j < x, when it encounters the malfunction. In response to the malfunction, the scientific instrument 302 can refrain from proceeding to a *(j* + 1)-th operation of the instrument workflow 402 and can instead create or throw the error message 404. In other words, the malfunction can be considered as causing the scientific instrument 302 to pause the instrument workflow 402, such that the instrument workflow 402 is left unfinished or incomplete.

In various aspects, the error message 404 can be any suitable electronic data exhibiting any suitable format, size, or dimensionality (e.g., can be one or more scalars, one or more vectors, one or more matrices, one or more tensors, one or more character strings, or any suitable combination thereof) that can be considered as conveying, indicating, or otherwise representing any suitable information, properties, characteristics, or attributes about or regarding whatever malfunction has interrupted the instrument workflow 402. As a non-limiting example, the error message 404 can be any suitable structured text or alphanumeric identifier that indicates that a respective constituent piece of hardware or software of the scientific instrument 302 has stopped functioning properly, without specifically indicating what is wrong with that constituent piece of hardware or software (e.g., a first alphanumeric identifier can uniquely indicate that an oven of the scientific instrument 302 has stopped functioning properly but might not indicate what specifically is wrong with the oven; a second alphanumeric identifier can uniquely indicate that an ion beam emitter of the scientific instrument 302 has stopped functioning properly but might not indicate what specifically is wrong with the ion beam emitter). As another non-limiting example, the error message 404 can be any suitable structured text or alphanumeric identifier that indicates that a respective constituent piece of hardware or software of the scientific instrument 302 has encountered a respective type, category, or class of malfunction (e.g., a first alphanumeric identifier can uniquely indicate that an oven of the scientific instrument 302 has overheated; a second alphanumeric identifier can uniquely indicate that the oven of the scientific instrument 302 has physically fractured). As yet another non-limiting example, the error message 404 can be any suitable unstructured text that describes that a respective constituent piece of hardware or software of the scientific instrument 302 has encountered a respective type, category, or class of malfunction (e.g., can be one or more natural language sentences or sentence fragments that semantically state or explain any suitable information regarding which part of the scientific instrument 302 has malfunctioned in which way). More generally, different constituent components of the scientific instrument 302 can fail or malfunction in different ways, and the error message 404 can represent or convey such information at any suitable level of specificity or generality (e.g., the error message 404 can indicate that the scientific instrument 302 has malfunctioned; the error message 404 can indicate that some specific part of the scientific instrument 302 has malfunctioned; or the error message 404 can indicate that some specific part of the scientific instrument 302 has experienced some specific type of malfunction).

In any case, the workflow component 320 can cause the scientific instrument 302 to begin performing the instrument workflow 402, and the error message 404 (and thus whatever underlying malfunction caused the error message 404) can be considered as interrupting the instrument workflow 402.

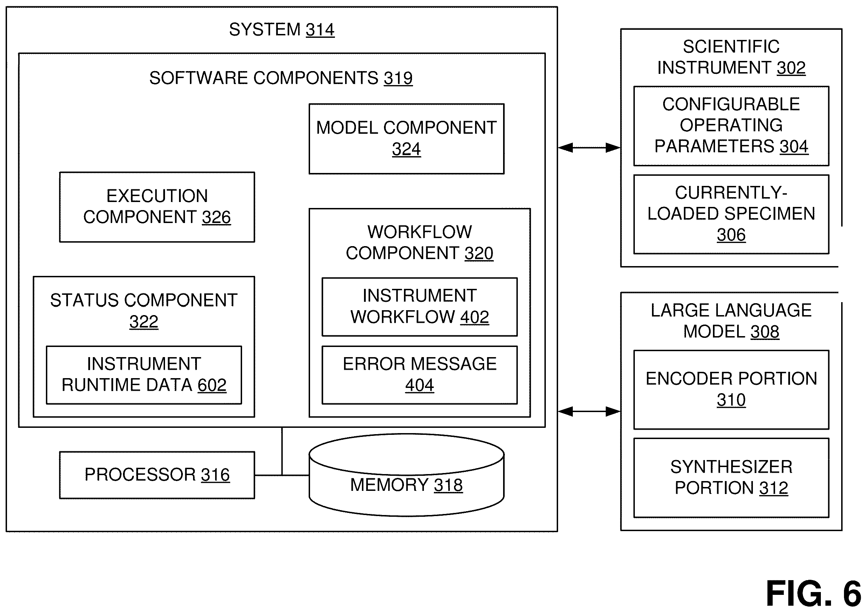

FIG. 6 illustrates a block diagram of an example, non-limiting system including instrument runtime data that can facilitate large language model resolution of scientific instrument workflow interruptions in accordance with one or more embodiments described herein.

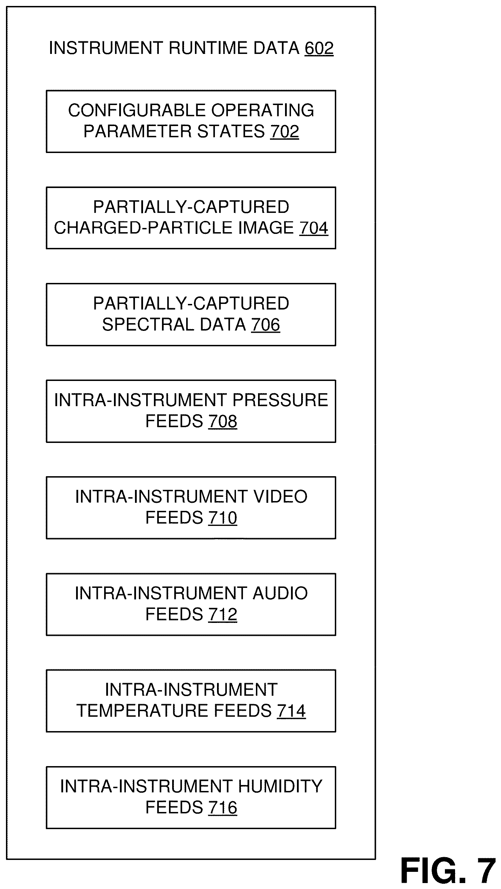

In various embodiments, the status component 322 can, in response to interruption of the instrument workflow 402 by the error message 404, electronically instruct, electronically command, or otherwise electronically cause the scientific instrument 302 to extract, record, or recall instrument runtime data 602. In various instances, the instrument runtime data 602 can be whatever electronic data that the scientific instrument 302 logged or otherwise utilized during or in connection with its partial-performance of the instrument workflow 402. Various non-limiting aspects are described with respect to FIG. 7.

FIG. 7 illustrates an example, non-limiting block diagram of the instrument runtime data 602 in accordance with one or more embodiments described herein.

In various embodiments, the instrument runtime data 602 can include a plurality of configurable operating parameter states 702. In various aspects, the plurality of configurable operating parameter states 702 can respectively correspond (e.g., in one-to-one fashion) to the plurality of configurable operating parameters 304. More specifically, each of the plurality of configurable operating parameter states 702 can be one or more scalars, one or more vectors, one or more matrices, one or more tensors, one or more character strings, or any suitable combination thereof that constitute or represent whatever state or value that a respective one of the plurality of configurable operating parameters 304 had or was assigned during the partial-performance of the instrument workflow 402. As a non-limiting example, the plurality of configurable operating parameter states 702 can indicate what focal spot size the scientific instrument 302 used when it was attempting the instrument workflow 402. As another non-limiting example, the plurality of configurable operating parameter states 702 can indicate what beam voltage the scientific instrument 302 used when it was attempting the instrument workflow 402. As even another non-limiting example, the plurality of configurable operating parameter states 702 can indicate what oven temperature the scientific instrument 302 used when it was attempting the instrument workflow 402.

In various embodiments in which the scientific instrument 302 is a charged-particle microscope and the instrument workflow 402 is an imaging protocol, the instrument runtime data 602 can include a partially-captured charged-particle image 704. In various aspects, the partially-captured charged-particle image 704 can be any suitable partially-filled or partially-generated pixel array or voxel array that the scientific instrument 302 created during its partial-performance of the instrument workflow 402. For instance, it can have been desired that the instrument workflow 402 would yield an SEM image depicting or illustrating the currently-loaded specimen 306. However, due to being interrupted by the error message 404, the instrument workflow 402 can instead have yielded a not-fully-formed SEM image having a large proportion of pixels that still have their default values (e.g., that are still at zero when they should not be), and that not-fully-formed SEM image can be considered as the partially-captured charged-particle image 704. In other words, the scientific instrument 302 can have been in the middle of rasterizing the currently-loaded specimen 306 when the instrument workflow 402 was interrupted, and so the partially-captured charged-particle image 704 can have large swaths of blank space representing whatever areas of the currently-loaded specimen 306 that were supposed or intended to be rasterized but that had not yet been rasterized. It must be emphasized that conventional techniques consider the partially-captured charged-particle image 704 to be wasted or corrupted and thus useless.

In various embodiments in which the scientific instrument 302 is a chromatograph or mass spectrometer and the instrument workflow 402 is a scanning or screening protocol, the instrument runtime data 602 can include partially-captured spectral data 706. In various aspects, the partially-captured spectral data 706 can be any suitable partially-filled or partially-generated chromatogram or mass spectrum that the scientific instrument 302 created during its partial-performance of the instrument workflow 402. For instance, it can have been desired that the instrument workflow 402 would yield for the currently-loaded specimen 306 a chromatograph (e.g., abundance versus retention time) and a respective mass spectrum (e.g., abundance versus mass-to-charge ratio) for each peak in the chromatogram, with a maximum retention time of 120 seconds being implemented. However, due to being interrupted by the error message 404, the instrument workflow 402 can instead have collected chromatographic and mass spectral data only for the first 60 seconds of retention time. Accordingly, whatever chromatogram and mass spectra that were yielded by the instrument workflow 402 would lack whatever chromatographic or ion peaks that would arise only at retention times greater than 60 seconds. Such incomplete or unfinished chromatogram or mass spectra can be collectively considered as the partially-captured spectral data 706. It must be emphasized that conventional techniques consider the partially-captured spectral data 706 to be wasted or corrupted and thus useless.

In various embodiments, the instrument runtime data 602 can include one or more intra-instrument pressure feeds 708. In particular, the scientific instrument 302 can, in various aspects, be outfitted or otherwise equipped with one or more integrated or built-in pressure sensors. Non-limiting examples of such integrated or built-in pressure sensors can include: strain gauges; capacitive pressure transducers; resistive pressure transducers; piezoelectric pressure transducers; or optical pressure transducers. In any case, each of such integrated or built-in pressure sensors can continually, continuously, or periodically monitor whatever solid or fluid pressure a respective hardware component of the scientific instrument 302 experiences or encounters. Accordingly, each of the one or more intra-instrument pressure feeds 708 can be whatever timeseries of pressure measurements was recorded by a respective integrated or built-in pressure sensor during the partial-performance of the instrument workflow 402.

In various embodiments, the instrument runtime data 602 can include one or more intra-instrument video feeds 710. In particular, the scientific instrument 302 can, in various aspects, be outfitted or otherwise equipped with one or more integrated or built-in video cameras. Non-limiting examples of such integrated or built-in video cameras can include: endoscopes; thermal imaging cameras; night vision cameras; or any suitable type of visible spectrum camera. In any case, each of such integrated or built-in video cameras can continually, continuously, or periodically monitor the visual appearance of a respective hardware component of the scientific instrument 302. Accordingly, each of the one or more intra-instrument video feeds 710 can be whatever timeseries of video frames was recorded by a respective integrated or built-in video camera during the partial-performance of the instrument workflow 402.

In various embodiments, the instrument runtime data 602 can include one or more intra-instrument audio feeds 712. In particular, the scientific instrument 302 can, in various aspects, be outfitted or otherwise equipped with one or more integrated or built-in microphones. Non-limiting examples of such integrated or built-in microphones can include: dynamic microphones; capacitor microphones; ribbon microphones; piezoelectric microphones; or electret microphones. In any case, each of such integrated or built-in microphones can continually, continuously, or periodically monitor the noises emitted by a respective hardware component of the scientific instrument 302. Accordingly, each of the one or more intra-instrument audio feeds 712 can be whatever timeseries of audio data was recorded by a respective integrated or built-in microphone during the partial-performance of the instrument workflow 402.

In various embodiments, the instrument runtime data 602 can include one or more intra-instrument temperature feeds 714. In particular, the scientific instrument 302 can, in various aspects, be outfitted or otherwise equipped with one or more integrated or built-in temperature sensors. Non-limiting examples of such integrated or built-in video temperature sensors can include: thermocouples; thermistors; semiconductor-based thermometers; thermal infrared sensors; or thermal diodes. In any case, each of such integrated or built-in temperature sensors can continually, continuously, or periodically monitor the temperatures experienced by a respective hardware component of the scientific instrument 302. Accordingly, each of the one or more intra-instrument temperature feeds 714 can be whatever timeseries of temperature measurements was recorded by a respective integrated or built-in temperature sensor during the partial-performance of the instrument workflow 402.

In various embodiments, the instrument runtime data 602 can include one or more intra-instrument humidity feeds 716. In particular, the scientific instrument 302 can, in various aspects, be outfitted or otherwise equipped with one or more integrated or built-in humidity sensors. Non-limiting examples of such integrated or built-in humidity sensors can include: capacitive hygroscopes; resistive hygroscopes; optical hygroscopes; gravimetric hygroscopes; piezoelectric hygroscopes; or solid-state hygroscopes. In any case, each of such integrated or built-in humidity sensors can continually, continuously, or periodically monitor the humidity experienced by a respective hardware component of the scientific instrument 302. Accordingly, each of the one or more intra-instrument humidity feeds 716 can be whatever timeseries of humidity measurements was recorded by a respective integrated or built-in humidity sensor during the partial-performance of the instrument workflow 402.

It should be understood or otherwise appreciated that FIG. 7 shows a mere non-limiting example of the instrument runtime data 602. In some cases, any of the plurality of configurable operating parameter states 702, the partially-captured charged-particle image 704, the partially-captured spectral data 706, the one or more intra-instrument pressure feeds 708, the one or more intra-instrument video feeds 710, the one or more intra-instrument audio feeds 712, the one or more intra-instrument temperature feeds 714, or the one or more intra-instrument humidity feeds 716 can be excluded or omitted from the instrument runtime data 602 based on data availability or otherwise as desired.

In any case, the status component 322 can electronically collect or retrieve the instrument runtime data 602 from the scientific instrument 302, in response to the instrument workflow 402 being interrupted by the error message 404.

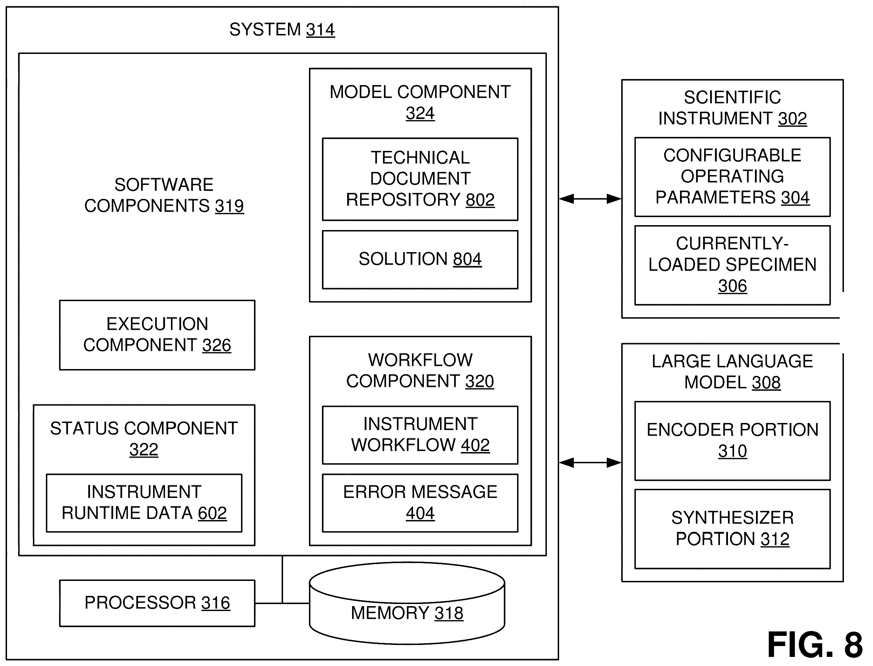

FIG. 8 illustrates a block diagram of an example, non-limiting system including a technical document repository and a solution that can facilitate large language model resolution of scientific instrument workflow interruptions in accordance with one or more embodiments described herein.

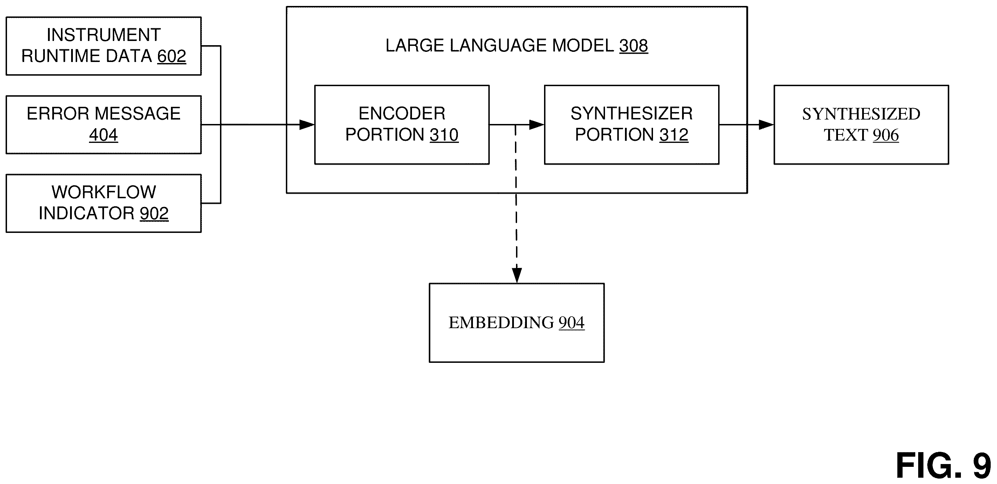

In various embodiments, the model component 324 can electronically store, electronically maintain, electronically control, or otherwise electronically access (e.g., locally or remotely) a technical document repository 802. In various aspects, the model component 324 can electronically generate a solution 804 to the error message 404, by leveraging the LLM 308, the instrument runtime data 602, or the technical document repository 802. Various on-limiting details are described with respect to FIGs. 9-14.

FIGs. 9-14 illustrate example, non-limiting block diagrams showing how the solution 804 to the error message 404 can be generated based on the instrument runtime data 602 and the technical document repository 802 in accordance with one or more embodiments described herein.

First, consider FIG. 9. In various cases, there can be a workflow indicator 902. In various instances, the workflow indicator 902 can be any suitable electronic data exhibiting any suitable format, size, or dimensionality (e.g., can be one or more scalars, one or more vectors, one or more matrices, one or more tensors, one or more character strings, or any suitable combination thereof) that can be considered as conveying, indicating, or otherwise representing any suitable information, properties, or attributes about or regarding the instrument workflow 402. As a non-limiting example, the workflow indicator 902 can be any suitable structured text or alphanumeric identifier that indicates or uniquely corresponds to an identity of the instrument workflow 402. In some instances, the workflow indicator 902 and the error message 404 can collectively be considered as indicating: how many total steps, actions, or operations are involved in the instrument workflow 402; and at which of those steps, actions, or operations the instrument workflow 402 was interrupted.

In various aspects, the model component 324 can electronically execute the LLM 308 on the instrument runtime data 602, on the error message 404, on the workflow indicator 902, or on any suitable combination thereof. In various instances, such execution can cause the LLM 308 to produce some synthesized text 906.

More specifically, the model component 324 can concatenate the instrument runtime data 602, the error message 404, and the workflow indicator 902 together. Note that, in some cases, such concatenation can comprise any other combination of the aforementioned (e.g., can comprise fewer than all of the instrument runtime data 602, the error message 404, and the workflow indicator 902). In various instances, the model component 324 can feed or route that concatenation to the input layer of the encoder portion 310. In various cases, that concatenation can complete a forward pass through the one or more hidden layers of the encoder portion 310. In various aspects, the output layer of the encoder portion 310 can compute or otherwise calculate an embedding 904, based on activation maps or feature maps provided by the one or more hidden layers of the encoder portion 310.

In various instances, the embedding 904 can be considered as a latent vector representation that the encoder portion 310 believes or infers corresponds to the concatenation of the instrument runtime data 602, the error message 404, or the workflow indicator 902. More specifically, the embedding 904 can be one or more scalars, one or more vectors, one or more matrices, one or more tensors, or any suitable combination thereof. In various aspects, the dimensionality of the embedding 904 (e.g., the total number or cardinality of numerical elements within the embedding 904) can be smaller (e.g., many orders of magnitude smaller, in some cases) than the total or cumulative dimensionality of the instrument runtime data 602, the error message 404, or the workflow indicator 902. In various instances, despite its smaller dimensionality, the embedding 904 can nevertheless be considered as representing, albeit in hidden or non-apparent fashion, at least some substantive or semantic content of the instrument runtime data 602, the error message 404, or the workflow indicator 902. In other words, the embedding 904 can be considered as a compact or compressed numerical representation of the instrument runtime data 602, the error message 404, or the workflow indicator 902. Note that the embedding 904 can be considered as representing the instrument runtime data 602, the error message 404, or the workflow indicator 902 in a latent, obscure, or otherwise hidden fashion, since a third-party that has no connection or relationship to the encoder portion 310 would be unable to recreate or guess the instrument runtime data 602, the error message 404, or the workflow indicator 902 from the embedding 904 alone.

Now, in various aspects, the embedding 904 can be fed or routed to the input layer of the synthesizer portion 312. In various cases, the embedding 904 can complete a forward pass through the one or more hidden layers of the synthesizer portion 312. In various aspects, the output layer of the synthesizer portion 312 can compute or otherwise calculate the synthesized text 906, based on activation maps or feature maps provided by the one or more hidden layers of the synthesizer portion 312.

In various aspects, the synthesized text 906 can be one or more declarative sentences or sentence fragments that the synthesizer portion 312 has generated based on the embedding 904. Note that the synthesized text 906 can be not an estimated or approximated reconstruction of the instrument runtime data 602, the error message 404, or the workflow indicator 902. Instead, the synthesized text 906 can be any suitable number of synthetic sentences that somehow semantically or substantively relate to the embedding 904 and thus to the instrument runtime data 602, the error message 404, or the workflow indicator 902. In some cases, the synthesized text 906 can be considered as containing hallucinations that are semantically or substantively related to the instrument runtime data 602, the error message 404, or the workflow indicator 902.

In various aspects, the model component 324 can ignore, discard, or delete the synthesized text 906. However, the model component 324 can record, preserve, store, or otherwise maintain the embedding 904. In other words, the model component 324 can extract the embedding 904 from the encoder portion 310 (e.g., from hidden layers of the LLM 308).

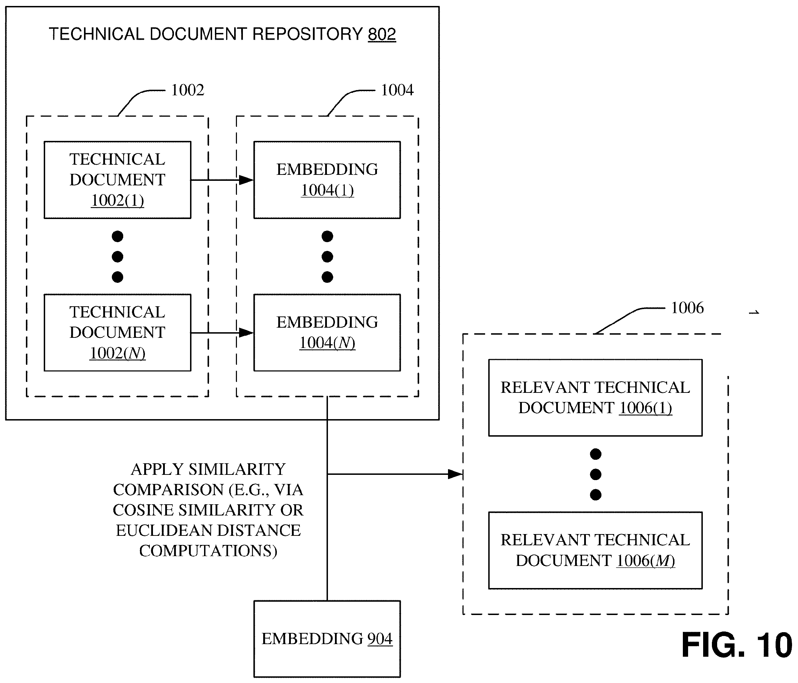

Now, consider FIG. 10. In various aspects, the technical document repository 802 can comprise a plurality of technical documents 1002. In various instances, the plurality of technical documents 1002 can comprise n documents, for any suitable positive integer n > 1: a technical document 1002(1) to a technical document *1002(n).* In various instances, each of the plurality of technical documents 1002 can be any suitable electronic file (e.g., word-doc file, PDF file, webpage file) that textually (or, in some cases, graphically or numerically) describes, teaches, shows, indicates, or otherwise conveys one or more technical features, details, or aspects of any suitable scientific instruments (which may or may not include the scientific instrument 302), or one or more technical features, details, or aspects of any suitable instrument workflows (which may or may not include the instrument workflow 402). As some non-limiting examples, any of the plurality of technical documents 1002 can be service manuals, maintenance handbooks, schematics, failure mode reports, or any portions thereof that describe or explain: the technological or scientific designs of any suitable charged-particle microscopes, chromatographs, or mass spectrometers (e.g., can list or show different constituent pieces, parts, or subsystems of various instruments and can describe how those constituent pieces, parts, or subsystems work); how to operate any suitable charged-particle microscopes, chromatographs, or mass spectrometers (e.g., can list or show different user-configurable settings or controls of various instruments and can explain what such settings or controls do); any suitable information regarding expected use or expected operation of any suitable charged-particle microscopes, chromatographs, or mass spectrometers (e.g., can show or explain various operating conditions or use-case scenarios that various instruments are designed to handle or are not designed to handle); any suitable information regarding how any suitable charged-particle microscopes, chromatographs, or mass spectrometers are expected or supposed to be maintained (e.g., can list, show, or explain various servicing tasks that are expected to be performed for various instruments); any suitable information regarding troubleshooting of any suitable charged-particle microscopes, chromatographs, or mass spectrometers (e.g., can describe or explain how to resolve various malfunction symptoms of various instruments); or any suitable information regarding workflows that are commonly performed by charged-particle microscopes, chromatographs, or mass spectrometers (e.g., can list, describe, or explain how different types of instrument workflows should be performed; can list, describe, or explain necessary or preparatory conditions that are required for different instrument workflows; can list, describe, or explain how different instrument workflows commonly fail; can list, describe, or explain how to rectify common problems of different instrument workflows). As some other non-limiting examples, any of the plurality of technical documents 1002 can be: any source code script or file associated with any suitable charged-particle microscopes, chromatographs, or mass spectrometers; any suitable maintenance tickets or servicing tickets associated with any suitable charged-particle microscopes, chromatographs, or mass spectrometers; or any suitable customer complaint forms or customer comment forms associated with any suitable charged-particle microscopes, chromatographs, or mass spectrometers.

It is to be appreciated and understood that any of the plurality of technical documents 1002 can be any sub-portion or sub-part of some larger document. For example, any of the plurality of technical documents 1002 can be a chapter, section, paragraph, or even sentence from some lengthier document.

In various aspects, the model component 324 can electronically generate a plurality of embeddings 1004, by executing the LLM 308 as described above on each of the plurality of technical documents 1002.

As a non-limiting example, the model component 324 can execute the LLM 308 on the technical document 1002(1), and the model component 324 can extract, during that execution, an embedding 1004(1) from the LLM 308. More specifically, the model component 324 can feed or route the technical document 1002(1) to the input layer of the encoder portion 310, the technical document 1002(1) can complete a forward pass through the one or more hidden layers of the encoder portion 310, and the output layer of the encoder portion 310 can compute or otherwise calculate the embedding 1004(1), based on activation maps or feature maps provided by the one or more hidden layers of the encoder portion 310. Note that the embedding 1004(1) can have the same format, size, or dimensionality as the embedding 904 (e.g., an embedding can be a uniform-dimensional or uniform-size vector representing a sentence; sentence-wise embeddings of a paragraph of sentences can be aggregated or averaged together to yield an embedding for the paragraph; paragraph-wise embeddings of a section or chapter can be aggregated or averaged together to yield an embedding for the section or chapter; section-wise or chapter-wise embeddings of an overall document can be aggregated or averaged together to yield an embedding for the overall document), and thus the embedding 1004(1) can be considered as a latent vector representation of the technical document 1002(1). In various cases, the embedding 1004(1) can then complete a forward pass through the synthesizer portion 312, but the model component 324 can ignore, disregard, or delete whatever synthesized textual content the synthesizer portion 312 creates based on the embedding 1004(1).

As another non-limiting example, the model component 324 can execute the LLM 308 on the technical document 1002(n), and the model component 324 can extract, during that execution, an embedding 1004(n) from the LLM 308. Indeed, just as described above, the model component 324 can feed or route the technical document 1002(*n)* to the input layer of the encoder portion 310, the technical document 1002(n) can complete a forward pass through the one or more hidden layers of the encoder portion 310, and the output layer of the encoder portion 310 can compute or otherwise calculate the embedding 1004(n), based on activation maps or feature maps provided by the one or more hidden layers of the encoder portion 310. So, the embedding 1004(*n*) can have the same format, size, or dimensionality as the embedding 904, and thus the embedding 1004(n) can be considered as a latent vector representation of the technical document 1002(n). As above, the embedding 1004(n) can then complete a forward pass through the synthesizer portion 312, but the model component 324 can ignore, disregard, or delete whatever synthesized textual content the synthesizer portion 312 creates based on the embedding 1004(n).

In various cases, the embedding 1004(1) to the embedding 1004(n) can collectively be considered as the plurality of embeddings 1004.

In various aspects, the model component 324 can electronically determine or identify a set of relevant technical documents 1006, by comparing the embedding 904 to the plurality of embeddings 1004. In particular, for each given embedding of the plurality of embeddings 1004, the model component 324 can compute any suitable error or similarity value between that given embedding and the embedding 904. As some non-limiting examples, such error or similarity value can involve: mean absolute error (MAE) computation; mean squared error (MSE) computation; cosine similarity computation; Euclidean distance computation; or cross-entropy computation. In any case, the model component 324 can conclude that the set of relevant technical documents 1006 are whichever of the plurality of technical documents 1002 whose embeddings (e.g., in 906) are most similar or closest to the embedding 904. As a non-limiting example, the model component 324 can identify whichever m of the plurality of technical documents 1002 have the most similar or closest embeddings to the embedding 904, and such m documents can be considered as the set of relevant technical documents 1006, for any suitable positive integer m. That is, the set of relevant technical documents 1006 can comprise m documents: a relevant technical document 1006(1) to a relevant technical document 1006(m). In other words, the relevant technical document 1006(1) can be whichever of the plurality of technical documents 1002 whose embedding is closest or most similar to the embedding 904, whereas the relevant technical document 1006(m) can be whichever of the plurality of technical documents 1002 whose embedding is the m-th closest or m-th most similar to the embedding 904.

In any case, the set of relevant technical documents 1006 can be considered as being substantively or semantically related in some way to the instrument runtime data 602, the error message 404, or the workflow indicator 902. As a non-limiting example, suppose that the workflow indicator 902 specifies that the instrument workflow 402 is a particular imaging protocol, and suppose that the error message 404 indicates that an ion beam emitter of the scientific instrument 302 has ceased functioning properly. In such case, any of the set of relevant technical documents 1006 can be structured or unstructured text that describes or explains: what constituent pieces or hardware or software make up the scientific instrument 302; what sequence of imaging operations make up the instrument workflow 402; how to perform, conduct, or implement each of those imaging operations; what focal spot size, raster pattern, or beam voltage the instrument workflow 402 necessitates; how ion beam emitters are designed, function, or are constructed; what types of malfunctions commonly afflict ion beam emitters; what types of malfunctions commonly result in partially-captured images, partially-captured spectral data, intra-instrument pressure measurements, intra-instrument video recordings, intra-instrument audio recordings, intra-instrument temperature measurements, or intra-instrument humidity measurements that are similar to those shown in the instrument runtime data 602; or how to troubleshoot or repair any of such malfunctions. Accordingly, the set of relevant technical documents 1006 can be considered as providing valuable contextual information regarding the instrument runtime data 602, the error message 404, or the workflow indicator 902.

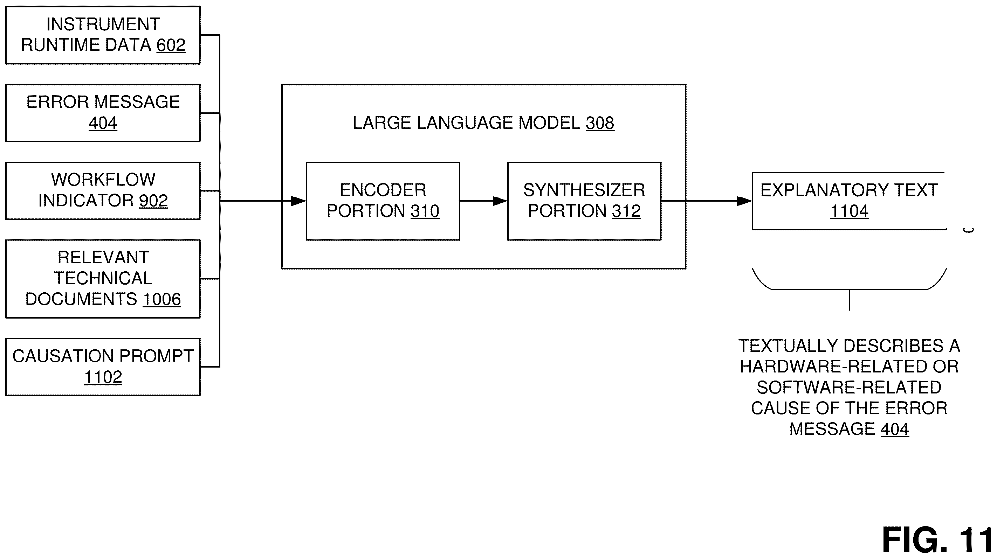

Next, consider FIG. 11. In various embodiments, there can be a causation prompt 1102. In various aspects, the causation prompt 1102 can be one or more unstructured or plain text sentences or sentence fragments that request or command that a specific hardware cause or software cause of whatever malfunction interrupted the instrument workflow 402 be identified, determined, or diagnosed. Indeed, the scientific instrument 302 can be made up of a multitude of constituent pieces of hardware or software (e.g., ion beam emitter, electron detector, collimating lens, vacuum pump, operating system, downloaded libraries); any given piece of hardware or software can be able to experience a multitude of types of failures or malfunctions (e.g., an ion beam emitter can experience a cathode failure, an anode failure, or an insulator failure); and a given type of failure or malfunction can have different underlying or root causes (e.g., an insulator failure of an ion beam emitter can be caused by: dielectric breakdown via voltage surges; dielectric breakdown via overheating; dielectric breakdown from vibratory fatigue; dielectric breakdown via moisture absorption; or dielectric breakdown via outgassing). So, the causation prompt 1102 can be considered as asking for identification of the specific, granular, underlying, or root cause of whatever malfunction interrupted the instrument workflow 402. As a non-limiting example, the causation prompt 1102 can be the following sentences: "Workflow specified by workflow indicator 902. Workflow interrupted by error message 404 and resulted in instrument runtime data 602. What specific malfunction caused the workflow to be interrupted?". As another non-limiting example, the causation prompt 1102 can be the following sentences: "Workflow specified by workflow indicator 902. Workflow interrupted by error message 404 and resulted in instrument runtime data 602. Diagnose the cause of the interruption."

So, in various instances, the model component 324 can electronically execute the LLM 308 on the instrument runtime data 602, on the error message 404, on the workflow indicator 902, on the set of relevant technical documents 1006, on the causation prompt 1102, or on any suitable combination thereof. In various cases, such execution can cause the LLM 308 to produce explanatory text 1104. More specifically, the model component 324 can concatenate the instrument runtime data 602, the error message 404, the workflow indicator 902, the set of relevant technical documents 1006, and the causation prompt 1102 together. As above, note that, in various cases, the concatenation can comprise any other combination of the aforementioned (e.g., can comprise fewer than all of the instrument runtime data 602, the error message 404, the workflow indicator 902, the set of relevant technical documents 1006, and the causation prompt 1102). In various instances, the model component 324 can feed that concatenation to the input layer of the encoder portion 310. In various aspects, that concatenation can complete a forward pass through the one or more hidden layers of the encoder portion 310. In various instances, the output layer of the encoder portion 310 can compute or otherwise calculate one or more embeddings (not shown), based on activation maps or feature maps provided by the one or more hidden layers of the encoder portion 310. In various cases, those one or more embeddings can be routed to the input layer of the synthesizer portion 312. In various aspects, those one or more embeddings can complete a forward pass through the one or more hidden layers of the synthesizer portion 312, and the output layer of the synthesizer portion 312 can compute or otherwise calculate the explanatory text 1104 based on activation maps or feature maps provided by the one or more hidden layers of the synthesizer portion 312.

In various aspects, the explanatory text 1104 can be one or more unstructured or plain text declarative sentences or sentence fragments that semantically answer the causation prompt 1102. That is, the explanatory text 1104 can be synthesized text that describes or states what underlying issue with the scientific instrument 302 seems to have caused the instrument workflow 402 to be interrupted. In other words, the explanatory text 1104 can be natural language or plain text that describes or explains what specific hardware-related problem or software-related problem forced the scientific instrument 302 to generate the error message 404 in the middle of the instrument workflow 402. In various instances, the workflow indicator 902 can be considered as indicating known information regarding the instrument workflow 402, whereas the error message 404 and the instrument runtime data 602 (as enhanced or supplemented by the set of relevant technical documents 1006) can be considered as measured or detected information regarding whatever as-yet-unknown malfunction or failure interrupted the instrument workflow 402. In some cases, that as-yet-unknown malfunction or failure can have left a trace, signature, or other unique influence on the instrument runtime data 602. After all, different underlying failures can manifest in different ways (e.g., some failures can manifest visually, which may uniquely affect the partially-captured charged-particle image 704 or the one or more intra-instrument video feeds 710; other failures can manifest aurally, which may uniquely affect the one or more intra-instrument audio feeds 712; yet other failures can manifest thermally, which may uniquely affect the one or more intra-instrument temperature feeds 714). So, the LLM 308 can be considered as leveraging the set of relevant technical documents 1006 so as to recognize whatever traces, signatures, or unique influences that as-yet-unknown malfunction has imparted in the instrument runtime data 602, and such recognition can thus allow an identity of that as-yet-unknown malfunction to be inferred. The explanatory text 1104 can thus semantically convey or indicate that identity.

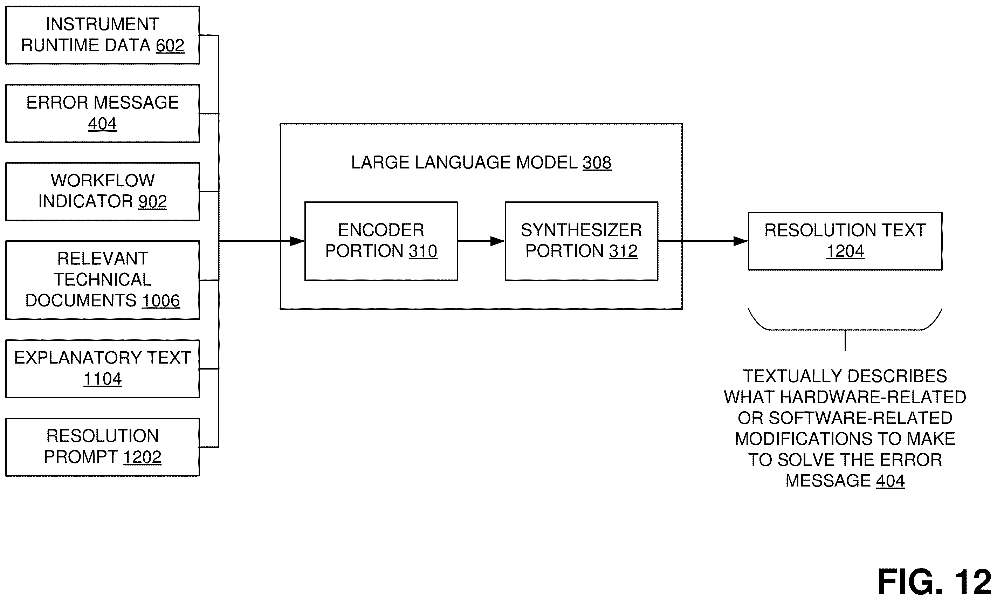

Now, consider FIG. 12. In various embodiments, there can be a resolution prompt 1202. In various aspects, the resolution prompt 1202 can be one or more unstructured or plain text sentences or sentence fragments that request or command that remedial actions for whatever specific malfunction is specified in the explanatory text 1104 be identified or determined. Indeed, as mentioned above, a given type of failure or malfunction can have different underlying or root causes, and different underlying or root causes can necessitate different remedial actions (e.g., dielectric breakdown via voltage surges can be repaired or addressed via a first sequence of troubleshooting or repair actions; in contrast, dielectric breakdown via overheating might not respond to the first sequence of troubleshooting or repair actions and might instead be repaired or addressed via a second sequence of troubleshooting or repair actions). So, the resolution prompt 1202 can be considered as asking for identification of specific actions or protocols that, if performed on the scientific instrument 302, would resolve or cure whatever caused the instrument workflow 402 to be interrupted. As a non-limiting example, the resolution prompt 1202 can be the following sentences: "Workflow specified by workflow indicator 902. Workflow interrupted by error message 404 and resulted in instrument runtime data 602. Inferred cause of interruption indicated by explanatory text 1104. How to solve the inferred cause?". As another non-limiting example, the resolution prompt 1202 can be the following sentences: "Workflow specified by workflow indicator 902. Workflow interrupted by error message 404 and resulted in instrument runtime data 602. Inferred cause of interruption indicated by explanatory text 1104. Determine how to fix."

So, in various instances, the model component 324 can electronically execute the LLM 308 on the instrument runtime data 602, on the error message 404, on the workflow indicator 902, on the set of relevant technical documents 1006, on the explanatory text 1104, on the resolution prompt 1202, or on any suitable combination thereof. In various cases, such execution can cause the LLM 308 to produce resolution text 1204. More specifically, the model component 324 can concatenate the instrument runtime data 602, the error message 404, the workflow indicator 902, the set of relevant technical documents 1006, the explanatory text 1104, and the resolution prompt 1202 together. As above, note that, in various cases, the concatenation can comprise any other combination of the aforementioned (e.g., can comprise fewer than all of the instrument runtime data 602, the error message 404, the workflow indicator 902, the set of relevant technical documents 1006, the explanatory text 1104, or the resolution prompt 1202). In various instances, the model component 324 can feed that concatenation to the input layer of the encoder portion 310. In various aspects, that concatenation can complete a forward pass through the one or more hidden layers of the encoder portion 310. In various instances, the output layer of the encoder portion 310 can compute or otherwise calculate one or more embeddings (not shown), based on activation maps or feature maps provided by the one or more hidden layers of the encoder portion 310. In various cases, those one or more embeddings can be routed to the input layer of the synthesizer portion 312. In various aspects, those one or more embeddings can complete a forward pass through the one or more hidden layers of the synthesizer portion 312, and the output layer of the synthesizer portion 312 can compute or otherwise calculate the resolution text 1204 based on activation maps or feature maps provided by the one or more hidden layers of the synthesizer portion 312.

In various aspects, the resolution text 1204 can be one or more unstructured or plain text declarative sentences or sentence fragments that semantically answer the resolution prompt 1202. That is, the resolution text 1204 can be synthesized text that describes or states what sequence of remedial actions or operations would resolve or cure whatever underlying issue with the scientific instrument 302 is specified in the explanatory text 1104. In other words, the resolution text 1204 can be natural language or plain text that forms a tutorial teaching what specific hardware modifications or what specific software modifications would, if made to the scientific instrument 302, fix or repair the specific hardware-related problem or software-related problem that interrupted the instrument workflow 402. Non-limiting examples of remedial hardware modifications can include: replacing a consumable fluid, cartridge, or constituent component of the scientific instrument 302; tightening or loosening a specific bolt or fastener of the scientific instrument 302; drying, wetting, or lubricating a specified surface or lens of the scientific instrument 302; leveling, reorienting, or otherwise repositioning the scientific instrument 302; or adding structural supports to the scientific instrument 302. Non-limiting examples of remedial software modifications can include: rebooting or restarting the scientific instrument 302; adjusting a specific one of the plurality of configurable operating parameters 304 to a specific value; or editing a source code file of the scientific instrument 302. In any of these cases, the LLM 308 can be considered as leveraging its own inference indicated in the explanatory text 1104 and whatever traces, signatures, or unique patterns are present in the instrument runtime data 602 (as supplemented or enhanced by the set of relevant technical documents 1006) so as to determine how to solve whatever caused the error message 404 to interrupt the instrument workflow 402. The resolution text 1204 can thus semantically convey or indicate that determination.

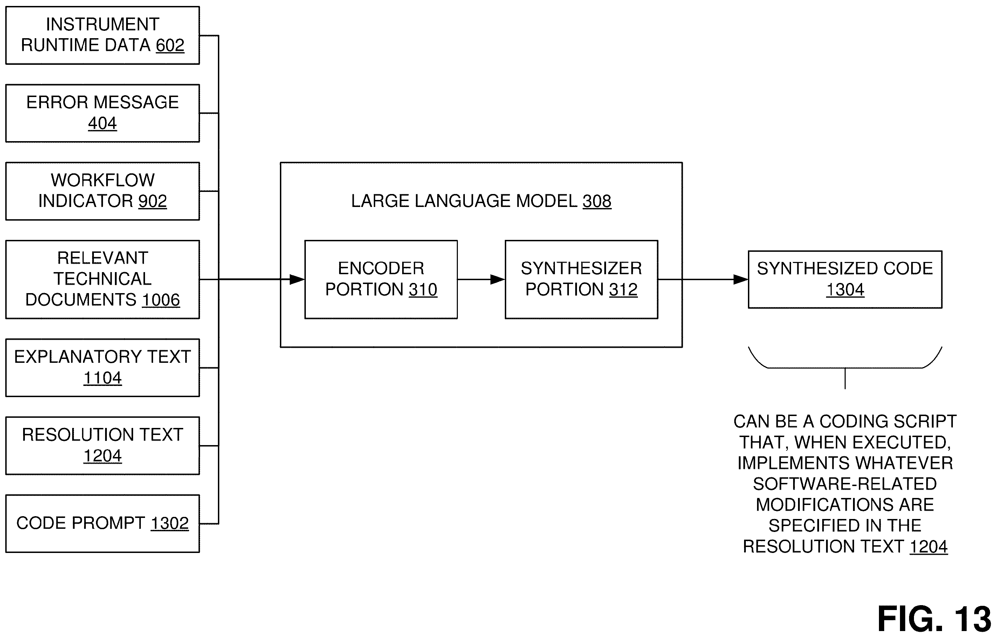

Next, consider FIG. 13. In various embodiments, there can be a code prompt 1302. In various aspects, the code prompt 1302 can be one or more unstructured or plain text sentences or sentence fragments that request or command that a coding script be generated or written so as to implement whatever software modifications that are specified in the resolution text 1204. As a non-limiting example, the code prompt 1302 can be the following sentences: "Workflow specified by workflow indicator 902. Workflow interrupted by error message 404 and resulted in instrument runtime data 602. Inferred cause of interruption indicated by explanatory text 1104. Resolution indicated in resolution text 1204. Write a script that implements the resolution".

So, in various instances, the model component 324 can electronically execute the LLM 308 on the instrument runtime data 602, on the error message 404, on the workflow indicator 902, on the set of relevant technical documents 1006, on the explanatory text 1104, on the resolution text 1204, on the code prompt 1302, or on any suitable combination thereof. In various cases, such execution can cause the LLM 308 to produce synthesized code 1304. More specifically, the model component 324 can concatenate the instrument runtime data 602, the error message 404, the workflow indicator 902, the set of relevant technical documents 1006, the explanatory text 1104, the resolution text 1204, and the code prompt 1302 together. As above, note that, in various cases, the concatenation can comprise any other combination of the aforementioned (e.g., can comprise fewer than all of the instrument runtime data 602, the error message 404, the workflow indicator 902, the set of relevant technical documents 1006, the explanatory text 1104, the resolution text 1204, or the code prompt 1302). In various instances, the model component 324 can feed that concatenation to the input layer of the encoder portion 310. In various aspects, that concatenation can complete a forward pass through the one or more hidden layers of the encoder portion 310. In various instances, the output layer of the encoder portion 310 can compute or otherwise calculate one or more embeddings (not shown), based on activation maps or feature maps provided by the one or more hidden layers of the encoder portion 310. In various cases, those one or more embeddings can be routed to the input layer of the synthesizer portion 312. In various aspects, those one or more embeddings can complete a forward pass through the one or more hidden layers of the synthesizer portion 312, and the output layer of the synthesizer portion 312 can compute or otherwise calculate the synthesized code 1304 based on activation maps or feature maps provided by the one or more hidden layers of the synthesizer portion 312.

In various aspects, the synthesized code 1304 can be one or more structured text that semantically addresses or satisfies the resolution prompt 1202. That is, the synthesized code 1304 can be a coding or programming script that is configured to, upon execution, perform or implement whatever remedial software modifications are specified in the resolution text 1204. In other words, the synthesized code 1304 can be one or more lines of computer-executable code (e.g., made up of variable definitions, function calls, for-loops, if-loops, or while-loops) that are written in any suitable programming syntax and that, when executed by a computing device, cause that computing device to perform whatever software-related actions that the LLM 308 has determined would solve whatever interrupted the instrument workflow 402.

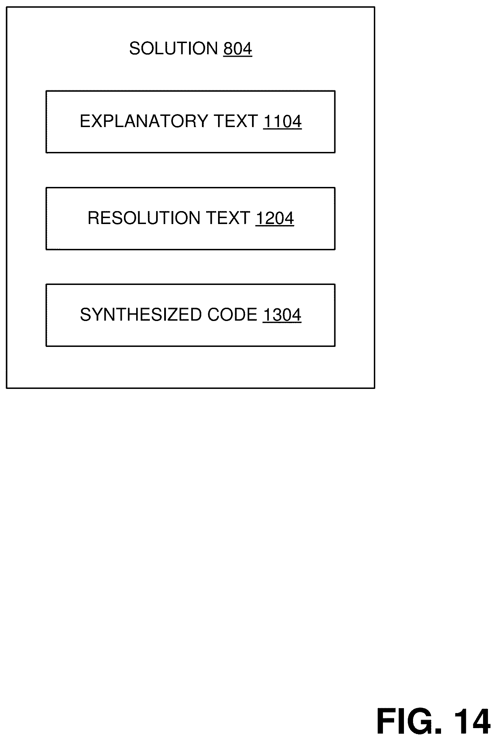

As shown in FIG. 14, the solution 804 can be considered as comprising or being made up of the explanatory text 1104, the resolution text 1204, and the synthesized code 1304.

It should be appreciated that, in some embodiments, the solution 804 can omit or exclude any of the explanatory text 1104, the resolution text 1204, or the synthesized code 1304.

Although FIGs. 9-14 depict the LLM 308 as sequentially generating the explanatory text 1104, the resolution text 1204, and the synthesized code 1304 one after the other, this is a mere non-limiting example for ease of explanation and illustration. In various aspects, there can be a single, unified prompt that contains any combination of the content of the causation prompt 1102, the resolution prompt 1202, or the code prompt 1302. In such cases, the LLM 308 can be executed on a concatenation of the instrument runtime data 602, the error message 404, the workflow indicator 902, the set of relevant technical documents 1006, and that unified prompt, and such execution can cause the LLM 308 to generate the explanatory text 1104, the resolution text 1204, and the synthesized code 1304 simultaneously or substantially simultaneously.

Although not explicitly shown in FIGs. 9-14, note that, in some embodiments in which the explanatory text 1104, the resolution text 1204, and the synthesized code 1304 are generated sequentially, the set of relevant technical documents 1006 can be iteratively or incrementally updated prior to each execution of the LLM 308. As a non-limiting example, the set of relevant technical documents 1006 can be initially extracted by performing an embedding search through the technical document repository 802 based on the embedding 904 as shown. However, after the explanatory text 1104 is generated, a new embedding can be created to collectively represent the instrument runtime data 602, the error message 404, the workflow indicator 902, and the explanatory text 1104, and that new embedding can be used to search through the technical document repository 802 again. Thus, whatever technical documents are retrieved from such new search can be considered as being semantically relevant not just to the instrument runtime data 602, the error message 404, and the workflow indicator 902, but also to the explanatory text 1104. These new relevant technical documents can thus be fed as inputs to the LLM 308 so as to increase the accuracy or the reliability of the resolution text 1204. Likewise, after the resolution text 1204 is generated, another new embedding can be created to collectively represent the instrument runtime data 602, the error message 404, the workflow indicator 902, the explanatory text 1104, and the resolution text 1204, and that another new embedding can be used to search through the technical document repository 802. So, whatever technical documents are retrieved from such another new search can be considered as being semantically relevant not just to the instrument runtime data 602, the error message 404, the workflow indicator 902, and the explanatory text 1104, but also to the resolution text 1204. These other new relevant technical documents can thus be fed as inputs to the LLM 308 so as to increase the accuracy or the reliability of the synthesized code 1304.

In any case, the model component 324 can generate the solution 804, by leveraging the LLM 308, the instrument runtime data 602, or the technical document repository 802.

In various embodiments, the execution component 326 can perform any suitable follow-on actions in response to generation of the solution 804. As a non-limiting example, the execution component 326 can electronically transmit the solution 804 (or any suitable portion thereof) to any suitable computing device. As another non-limiting example, the execution component 326 can electronically render the solution 804 (or any suitable portion thereof) on any suitable electronic computer screen or display.

It should be appreciated that the solution 804 can range in difficulty-of-implementation from easy to intensive. After all, some types of malfunctions can be easy to address (e.g., changing a small number of operating parameter values, cleaning a particular surface of the scientific instrument 302). In contrast, other types of malfunctions can be very difficult to address (e.g., heavily editing source code of the scientific instrument 302; heavily refurbishing or redesigning the physical structure of the scientific instrument 302). Accordingly, in situations where the solution 804 is easy or not intensive to implement, it can be acceptable or appropriate for the execution component 326 to electronically transmit or render the solution 804 to or on a computing device that is associated with an end-user of the scientific instrument 302. On the other hand, in situations where the solution 804 is intensive or not easy to implement, it can be acceptable or appropriate for the execution component 326 to electronically transmit or render the solution 804 to or on a computing device that is associated with an engineer or technical specialist of a manufacturer of the scientific instrument 302.

In any case, the solution 804 can be generated in mere seconds or minutes after the instrument workflow 402 is interrupted. Accordingly, the solution 804 can be considered as significantly expediting the process of troubleshooting such interruption, in comparison to the hours, days, weeks, or months needed by existing techniques.

In some embodiments in which the solution 804 contains the synthesized code 1304, the execution component 326 can electronically instruct, electronically command, or otherwise electronically cause the scientific instrument 302 to execute the synthesized code 1304. Thus, the scientific instrument 302 can perform whatever remedial software modifications are specified in the resolution text 1204. In response to such execution, the execution component 326 can, in some instances, electronically instruct, electronically command, or otherwise electronically cause the scientific instrument 302 to resume or retry the instrument workflow 402, so as to verify whether or not the underlying or root problem specified in the explanatory text 1104 has been successfully repaired by execution of the synthesized code 1304. If the scientific instrument 302 encounters the same or another interruption during such resumption or retrying of the instrument workflow 402, the system 314 can: repeat various actions described herein; or can electronically contact a technical specialist with an automated request for troubleshooting assistance.

In some embodiments, the LLM 308 can infer or predict that whatever malfunction that has caused the error message 404 has never been seen or encountered before. In such case, the explanatory text 1104 can indicate that the underlying cause of the error message 404 is unknown or never-before-seen. In such situations, the execution component 326 can, in response to the explanatory text 1104 indicating that the underlying cause is unknown or never-before-seen, electronically transmit a request for assistance to any suitable computing device of a technical specialist associated with the manufacturer of the scientific instrument 302. In some of such cases, the model component 324 can refrain from generating the resolution text 1204 and the synthesized code 1304. In others of such cases, the model component 324 can generate the resolution text 1204 and the synthesized code 1304, they can collectively be considered as potential or proposed solutions to the never-before-seen cause of the error message 404, and the execution component 326 can accordingly transmit them to the computing device of the technical specialist, thereby aiding the technical specialist notwithstanding the never-before-seen nature of the underlying cause of the error message 404.

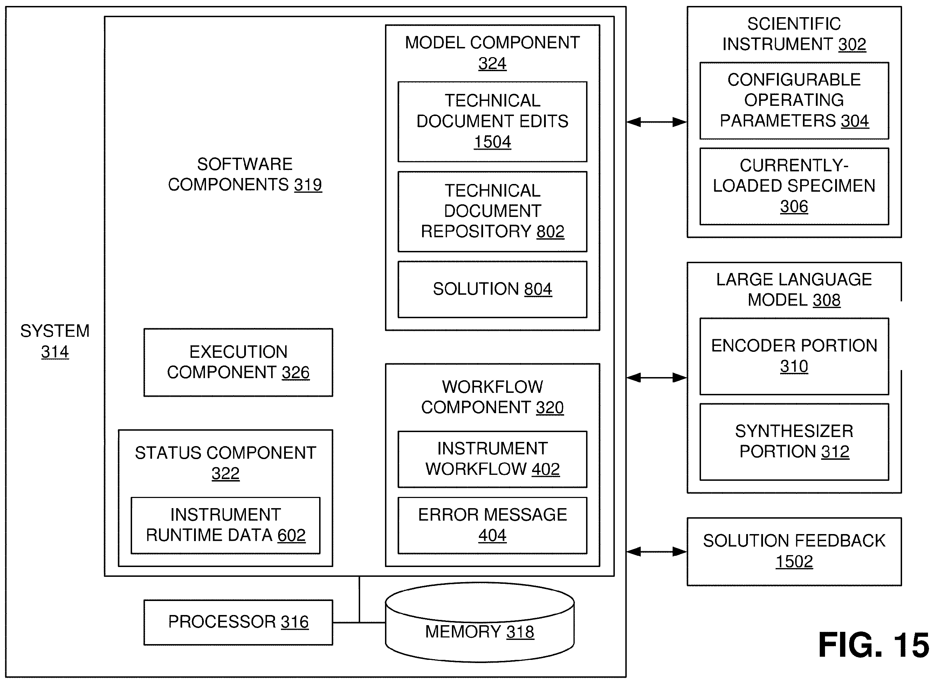

FIG. 15 illustrates a block diagram of an example, non-limiting system including solution feedback and a set of technical document edits that can facilitate large language model resolution of scientific instrument workflow interruptions in accordance with one or more embodiments described herein.

In various embodiments, the system 314 can electronically receive, electronically retrieve, or otherwise electronically access, from any suitable source, solution feedback 1502. In various aspects, the solution feedback 1502 can be any suitable electronic data (e.g., one or more scalars, one or more vectors, one or more matrices, one or more tensors, or one or more character strings) that represents or otherwise conveys shortcomings or inadequacies of the solution 804.

As a non-limiting example, the execution component 326 can, as mentioned above, share or render the solution 804 to or on a computing device associated with a technical specialist of the manufacturer of the scientific instrument 302. Thus, the technical specialist can be able to read or review the solution 804. In some cases, the technical specialist can identify one or more inaccurate or suboptimal portions of the solution 804 (e.g., can determine that the explanatory text 1104 recites an incorrect underlying cause; can determine that the resolution text 1204 recites an incorrect remedial action or an incorrect order of remedial actions; can determine that the synthesized code 1304 recites an incorrect function call). So, the solution feedback 1502 can be one or more declarative, natural language sentences or sentence fragments that are written by the technical specialist (e.g., using any suitable word processing software) and that describe or explain how or why one or more specific portions of the solution 804 are inaccurate.

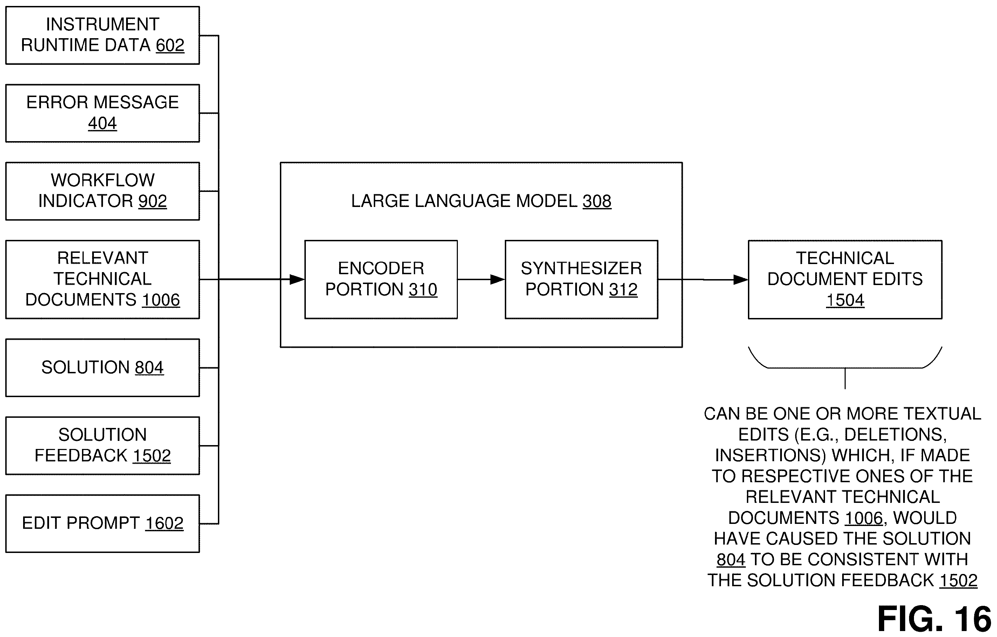

In various aspects, the model component 324 can leverage the LLM 308 and the solution feedback 1502, so as to generate one or more technical document edits 1504. Various non-limiting aspects are described with respect to FIG. 16.

FIG. 16 illustrates an example, non-limiting block diagram showing how the one or more technical document edits 1504 can be generated based on the solution feedback 1502 in accordance with one or more embodiments described herein.

In various embodiments, there can be an edit prompt 1602. In various aspects, the edit prompt 1602 can be one or more unstructured or plain text sentences or sentence fragments that request or command that respective ones of the set of relevant technical documents 1006 be edited so as to avoid whatever inferencing inaccuracies are specified in the solution feedback 1502. In other words, the LLM 308 can be considered as having generated the inferencing inaccuracies specified in the solution feedback 1502 due to having been misled by certain portions (e.g., certain chapters, certain pages, certain paragraphs, certain sentences) of the set of relevant technical documents 1006 (e.g., possibly due to those documents being out-of-date), and the edit prompt 1602 can be considered as asking the LLM 308 to identify those certain portions of the set of relevant technical documents 1006 and to determine how those certain portions should be edited or changed such that the LLM 308 is not misled in the same way again in the future. As a non-limiting example, the edit prompt 1602 can be the following sentences: "Workflow specified by workflow indicator 902. Workflow interrupted by error message 404 and resulted in instrument runtime data 602. Inferred troubleshooting indicated by solution 804 and based on set of relevant technical documents 1006. Solution inaccuracies indicated by solution feedback 1502. What edits to the set of relevant technical documents 1006 would have avoided the solution inaccuracies?". As a non-limiting example, the edit prompt 1602 can be the following sentences: "Workflow specified by workflow indicator 902. Workflow interrupted by error message 404 and resulted in instrument runtime data 602. Inferred troubleshooting indicated by solution 804 and based on set of relevant technical documents 1006. What edits would make the set of relevant technical documents 1006 consistent with the solution feedback 1502?".

So, in various instances, the model component 324 can electronically execute the LLM 308 on the instrument runtime data 602, on the error message 404, on the workflow indicator 902, on the set of relevant technical documents 1006, on the solution 804, on the solution feedback 1502, on the edit prompt 1602, or on any suitable combination thereof. In various cases, such execution can cause the LLM 308 to produce the one or more technical document edits 1504. More specifically, the model component 324 can concatenate the instrument runtime data 602, the error message 404, the workflow indicator 902, the set of relevant technical documents 1006, the solution 804, the solution feedback 1502, and the edit prompt 1602 together. As above, note that, in various cases, the concatenation can comprise any other combination of the aforementioned (e.g., can comprise fewer than all of the instrument runtime data 602, the error message 404, the workflow indicator 902, the set of relevant technical documents 1006, the solution 804, the solution feedback 1502, or the edit prompt 1602 together). In various instances, the model component 324 can feed that concatenation to the input layer of the encoder portion 310. In various aspects, that concatenation can complete a forward pass through the one or more hidden layers of the encoder portion 310. In various instances, the output layer of the encoder portion 310 can compute or otherwise calculate one or more embeddings (not shown), based on activation maps or feature maps provided by the one or more hidden layers of the encoder portion 310. In various cases, those one or more embeddings can be routed to the input layer of the synthesizer portion 312. In various aspects, those one or more embeddings can complete a forward pass through the one or more hidden layers of the synthesizer portion 312, and the output layer of the synthesizer portion 312 can compute or otherwise calculate the one or more technical document edits 1504 based on activation maps or feature maps provided by the one or more hidden layers of the synthesizer portion 312.

In various aspects, the one or more technical document edits 1504 can be one or more pieces of structured or unstructured text that semantically answer or address the edit prompt 1602. That is, the one or more technical document edits 1504 can be synthesized text that describes, states, or otherwise is one or more textual edits or textual changes (e.g., word deletions or insertions; sentence deletions, insertions, or rephrases; paragraph deletions, insertions, or rephrases) which, if made to respective ones of the set of relevant technical documents 1006, would have caused the solution 804 to be consistent with the solution feedback 1502. In other words, each of the one or more technical document edits 1504 can be a respective grammatical or semantic change, mark-up, or redline to a respective one of the set of relevant technical documents 1006, which change, mark-up, or redline would, if made to the relevant technical document prior to generation of the solution 804, have prevented the solution 804 from containing whatever inaccuracies are specified in the solution feedback 1502. In still other words, the LLM 308 can be considered as inferring which specific portions of which specific technical documents that it relied upon to generate the solution 804 caused the LLM 308 to be misled or to otherwise generate whatever incorrect inferences are specified in the solution feedback 1502, and the LLM 308 can further infer how those specific portions of those specific technical documents should be rewritten so as to prevent the LLM 308 from being similarly misled in the future. The one or more technical document edits 1504 can thus be considered as being the rewritten versions of (or as otherwise being rewriting instructions for) those specific portions of those specific technical documents.

In various embodiments, the execution component 326 can electronically implement the one or more technical document edits 1504. In other words, the execution component 326 can electronically apply the one or more technical document edits 1504 to respective ones of the set of relevant technical documents 1006. In still other words, the execution component 326 can semantically rewrite or change one or more of the set of relevant technical documents 1006 in whatever ways are specified by the one or more technical document edits 1504. Accordingly, the substantive information contained within the technical document repository 802 can be considered as being updated, and so troubleshooting solutions inferred by the LLM 308 in the future can have a higher likelihood of lacking inaccuracies.

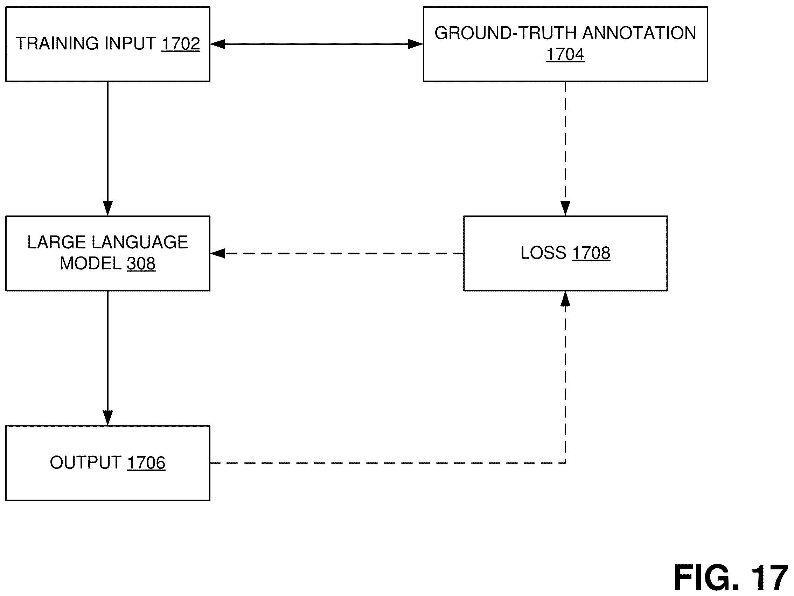

In order to increase the likelihood of the solution 804 being accurate or correct in accordance with various embodiments described herein, the LLM 308 can first undergo training. A non-limiting example of such training is described with respect to FIG. 17.

FIG. 17 illustrates an example, non-limiting block diagram showing how the LLM 308 can be trained in accordance with one or more embodiments described herein.

In various aspects, prior to beginning training, the trainable internal parameters (e.g., convolutional kernels, weight matrices, bias values) of the LLM 308 can be initialized in any suitable fashion (e.g., via random initialization) by the system 314.

In various embodiments, there can be a training input 1702 and a ground-truth annotation 1704. In various aspects, the training input 1702 can be any suitable textual, numerical, or graphical data that can be received by the LLM 308 as described herein. As some mere non-limiting examples, the training input 1702 can be any suitable combination of instrument runtime data, error messages, workflow indicators, technical documents, prompts, any other suitable structured or unstructured textual or numerical data, or any suitable combinations or concatenations thereof. In various instances, the ground-truth annotation 1704 can be whatever correct or accurate synthesized textual content (e.g., such as 1104, 1204, or 1504) or code (e.g., such as 1304) that is known or deemed to correspond to the training input 1702.

In any case, the system 314 can cause the LLM 308 to be executed on the training input 1702, thereby causing the LLM 308 to produce an output 1706. More specifically, in some cases, the training input 1702 can be fed or routed to the input layer of the LLM 308, the training input 1702 can complete a forward pass through the one or more hidden layers of the LLM 308, and the output layer of the LLM 308 can compute the output 1706 based on activation maps or feature maps provided by the one or more hidden layers of the LLM 308.

Note that the format, size, or dimensionality of the output 1706 can be dictated by the number, arrangement, sizes, or other characteristics of the neurons, convolutional kernels, attention blocks, or other internal parameters of the output layer (or of any other layers) of the LLM 308. Accordingly, the output 1706 can be forced to have any desired format, size, or dimensionality, by adding, removing, or otherwise adjusting characteristics of the output layer (or of any other layers) of the LLM 308.

In various aspects, the output 1706 can be considered as the predicted or inferred textual content (e.g., predicted or inferred explanatory text, predicted or inferred resolution text, predicted or inferred synthesized code, predicted or inferred technical document edits) that the LLM 308 has synthesized based on the training input 1702. In contrast, the ground-truth annotation 1704 can be considered as whatever correct or accurate textual content (e.g., correct or accurate explanatory text, correct or accurate resolution text, correct or accurate synthesized code, correct or accurate technical document edits) that is known or deemed to correspond to the training input 1702. Note that, if the LLM 308 has so far undergone no or little training, then the output 1706 can be highly inaccurate. In other words, the output 1706 can be very different from the ground-truth annotation 1704.

In various aspects, a loss 1708 (e.g., MAE, MSE, cross-entropy error) between the output 1706 and the ground-truth annotation 1704 can be computed by the system 314. In various instances, the trainable internal parameters of the LLM 308 can be incrementally updated via backpropagation (e.g., stochastic gradient descent) based on the loss 1708.

In various cases, such execution-and-update procedure can be repeated for any suitable number input-annotation pairs. This can ultimately cause the trainable internal parameters of the LLM 308 to become iteratively optimized for accurately performing text synthesis or code synthesis. In various aspects, any suitable training batch sizes, any suitable error/loss functions, or any suitable training termination criteria can be utilized during such training.

Although the herein disclosure mainly describes the LLM 308 as being trained in supervised fashion, this is a mere non-limiting example for ease of explanation and illustration. In various embodiments, any other suitable training paradigms can be used to train the LLM 308, such as unsupervised training, semi-supervised training, or reinforcement learning, any of which may be federated or unfederated.

Although the herein disclosure mainly describes the LLM 308 as being trained or configured to synthesize the solution 804, these are mere non-limiting examples for ease of illustration and explanation. In various embodiments, the LLM 308 can be configured to synthesize any suitable visual graphics that can supplement the solution 804. Non-limiting examples of such visual graphics can include: formatted reports or presentation slides that are based on, that are derived from, or that expound upon the solution 804; or plots, graphs, or charts that are based on, that are derived from, or that expound upon the solution 804. Accordingly, the execution component 326 can visually render such visual graphics in addition to or otherwise in conjunction with the solution 804.

Although the herein disclosure mainly describes the execution component 326 as visually or audibly rendering or presenting the solution 804 (or any associated visual graphics) to the user or technician of the scientific instrument 302, these are mere non-limiting examples for ease of explanation and illustration. In various embodiments, the execution component 326 can electronically transmit the solution 804 (or any associated visual graphics) to any suitable computing device that is associated with the scientific instrument 302. As a non-limiting example, the execution component 326 can share the solution 804 (or any associated visual graphics) with any suitable downstream software tools or applications that operate for or in conjunction with the scientific instrument 302 (e.g., some embodiments can involve sending the solution 804 to such downstream software tools or applications rather than presenting the solution 804 to the user or technician).

In various instances, machine learning algorithms or models can be implemented in any suitable way to facilitate any suitable aspects described herein. To facilitate some of the above-described machine learning aspects of various embodiments, consider the following discussion of artificial intelligence (Al). Various embodiments described herein can employ artificial intelligence to facilitate automating one or more features or functionalities. The components can employ various Al-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, z = (z₁, z₂, z₃, z₄, *zₙ),* to a confidence that the input belongs to a class, as by f(z) = *confidence*(*class*). Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the non-triggering events. Intuitively, this makes the classification correct for testing data that is near, but not identical to training data. Other directed and undirected model classification approaches include, e.g., naive Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

In order to provide additional context for various embodiments described herein, FIG. 18 and the following discussion are intended to provide a brief, general description of a suitable computing environment 1800 in which the various embodiments of the embodiment described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 18, the example environment 1800 for implementing various embodiments of the aspects described herein includes a computer 1802, the computer 1802 including a processing unit 1804, a system memory 1806 and a system bus 1808. The system bus 1808 couples system components including, but not limited to, the system memory 1806 to the processing unit 1804. The processing unit 1804 can be any of various commercially available processors. Dual microprocessors and other multi-processor architectures can also be employed as the processing unit 1804.

The system bus 1808 can be any of several types of bus structure that can further interconnect to a memory bus (with or without a memory controller), a peripheral bus, and a local bus using any of a variety of commercially available bus architectures. The system memory 1806 includes ROM 1810 and RAM 1812. A basic input/output system (BIOS) can be stored in a non-volatile memory such as ROM, erasable programmable read only memory (EPROM), EEPROM, which BIOS contains the basic routines that help to transfer information between elements within the computer 1802, such as during startup. The RAM 1812 can also include a high-speed RAM such as static RAM for caching data.

The computer 1802 further includes an internal hard disk drive (HDD) 1814 (e.g., EIDE, SATA), one or more external storage devices 1816 (e.g., a magnetic floppy disk drive (FDD) 1816, a memory stick or flash drive reader, a memory card reader, etc.) and a drive 1820, e.g., such as a solid state drive, an optical disk drive, which can read or write from a disk 1822, such as a CD-ROM disc, a DVD, a BD, etc. Alternatively, where a solid state drive is involved, disk 1822 would not be included, unless separate. While the internal HDD 1814 is illustrated as located within the computer 1802, the internal HDD 1814 can also be configured for external use in a suitable chassis (not shown). Additionally, while not shown in environment 1800, a solid state drive (SSD) could be used in addition to, or in place of, an HDD 1814. The HDD 1814, external storage device(s) 1816 and drive 1820 can be connected to the system bus 1808 by an HDD interface 1824, an external storage interface 1826 and a drive interface 1828, respectively. The interface 1824 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 1802, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1812, including an operating system 1830, one or more application programs 1832, other program modules 1834 and program data 1836. All or portions of the operating system, applications, modules, or data can also be cached in the RAM 1812. The systems and methods described herein can be implemented utilizing various commercially available operating systems or combinations of operating systems.

Computer 1802 can optionally comprise emulation technologies. For example, a hypervisor (not shown) or other intermediary can emulate a hardware environment for operating system 1830, and the emulated hardware can optionally be different from the hardware illustrated in FIG. 18. In such an embodiment, operating system 1830 can comprise one virtual machine (VM) of multiple VMs hosted at computer 1802. Furthermore, operating system 1830 can provide runtime environments, such as the Java runtime environment or the .NET framework, for applications 1832. Runtime environments are consistent execution environments that allow applications 1832 to run on any operating system that includes the runtime environment. Similarly, operating system 1830 can support containers, and applications 1832 can be in the form of containers, which are lightweight, standalone, executable packages of software that include, e.g., code, runtime, system tools, system libraries and settings for an application.

Further, computer 1802 can be enable with a security module, such as a trusted processing module (TPM). For instance with a TPM, boot components hash next in time boot components, and wait for a match of results to secured values, before loading a next boot component. This process can take place at any layer in the code execution stack of computer 1802, e.g., applied at the application execution level or at the operating system (OS) kernel level, thereby enabling security at any level of code execution.

A user can enter commands and information into the computer 1802 through one or more wired/wireless input devices, e.g., a keyboard 1838, a touch screen 1840, and a pointing device, such as a mouse 1842. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 1804 through an input device interface 1844 that can be coupled to the system bus 1808, but can be connected by other interfaces, such as a parallel port, an IEEE 1394 serial port, a game port, a USB port, an IR interface, a BLUETOOTH^{®} interface, etc.

A monitor 1846 or other type of display device can be also connected to the system bus 1808 via an interface, such as a video adapter 1848. In addition to the monitor 1846, a computer typically includes other peripheral output devices (not shown), such as speakers, printers, etc.

The computer 1802 can operate in a networked environment using logical connections via wired or wireless communications to one or more remote computers, such as a remote computer(s) 1850. The remote computer(s) 1850 can be a workstation, a server computer, a router, a personal computer, portable computer, microprocessor-based entertainment appliance, a peer device or other common network node, and typically includes many or all of the elements described relative to the computer 1802, although, for purposes of brevity, only a memory/storage device 1852 is illustrated. The logical connections depicted include wired/wireless connectivity to a local area network (LAN) 1854 or larger networks, e.g., a wide area network (WAN) 1856. Such LAN and WAN networking environments are commonplace in offices and companies, and facilitate enterprise-wide computer networks, such as intranets, all of which can connect to a global communications network, e.g., the Internet.

When used in a LAN networking environment, the computer 1802 can be connected to the local network 1854 through a wired or wireless communication network interface or adapter 1858. The adapter 1858 can facilitate wired or wireless communication to the LAN 1854, which can also include a wireless access point (AP) disposed thereon for communicating with the adapter 1858 in a wireless mode.

When used in a WAN networking environment, the computer 1802 can include a modem 1860 or can be connected to a communications server on the WAN 1856 via other means for establishing communications over the WAN 1856, such as by way of the Internet. The modem 1860, which can be internal or external and a wired or wireless device, can be connected to the system bus 1808 via the input device interface 1844. In a networked environment, program modules depicted relative to the computer 1802 or portions thereof, can be stored in the remote memory/storage device 1852. It will be appreciated that the network connections shown are example and other means of establishing a communications link between the computers can be used.

When used in either a LAN or WAN networking environment, the computer 1802 can access cloud storage systems or other network-based storage systems in addition to, or in place of, external storage devices 1816 as described above, such as but not limited to a network virtual machine providing one or more aspects of storage or processing of information. Generally, a connection between the computer 1802 and a cloud storage system can be established over a LAN 1854 or WAN 1856 e.g., by the adapter 1858 or modem 1860, respectively. Upon connecting the computer 1802 to an associated cloud storage system, the external storage interface 1826 can, with the aid of the adapter 1858 or modem 1860, manage storage provided by the cloud storage system as it would other types of external storage. For instance, the external storage interface 1826 can be configured to provide access to cloud storage sources as if those sources were physically connected to the computer 1802.

The computer 1802 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a predefined structure as with a conventional network or simply an ad hoc communication between at least two devices.

FIG. 19 is a schematic block diagram of a sample computing environment 1900 with which the disclosed subject matter can interact. The sample computing environment 1900 includes one or more client(s) 1910. The client(s) 1910 can be hardware or software (e.g., threads, processes, computing devices). The sample computing environment 1900 also includes one or more server(s) 1930. The server(s) 1930 can also be hardware or software (e.g., threads, processes, computing devices). The servers 1930 can house threads to perform transformations by employing one or more embodiments as described herein, for example. One possible communication between a client 1910 and a server 1930 can be in the form of a data packet adapted to be transmitted between two or more computer processes. The sample computing environment 1900 includes a communication framework 1950 that can be employed to facilitate communications between the client(s) 1910 and the server(s) 1930. The client(s) 1910 are operably connected to one or more client data store(s) 1920 that can be employed to store information local to the client(s) 1910. Similarly, the server(s) 1930 are operably connected to one or more server data store(s) 1940 that can be employed to store information local to the servers 1930.

An example, non-limiting apparatus for performing various embodiments described herein is shown in FIG. 20. FIG. 20 illustrates a non-limiting example of a dual beam system 2010 with a vertically mounted scanning electron microscope (SEM) column and a focused ion beam (FIB) column mounted at an angle of approximately 52 degrees from the vertical. Such dual beam systems are commercially available, for example, from FEI Company, Hillsboro, Oregon, the assignee of the present application. While FIG. 20 shows an example of suitable microscopy hardware with which various embodiments described herein can be implemented, it is to be appreciated that such microscopy hardware is non-limiting. In other words, various embodiments described herein can be implemented in conjunction with any other suitable types of microscopy hardware. The dual beam system 2010 is a non-limiting example of the scientific instrument 302 or of any other scientific instruments discussed above.

A scanning electron microscope 2041, along with a power supply and control unit 2045, can be provided with the dual beam system 2010. An electron beam 2043 can be emitted from a cathode 2052 by applying voltage between the cathode 2052 and an anode 2054. The electron beam 2043 can be focused to a fine spot by means of a condensing lens 2056 and an objective lens 2058. The electron beam 2043 can be scanned two-dimensionally on any suitable specimen by means of a deflection coil 2060. Operation of the condensing lens 2056, the objective lens 2058, or the deflection coil 2060 can be controlled by the power supply and control unit 2045.

The electron beam 2043 can be focused onto a substrate 2022, which can be on a movable X-Y stage 2025 within a lower chamber 2026. When the electrons in the electron beam 2043 strike the substrate 2022, secondary electrons can be emitted. These secondary electrons can be detected by a secondary electron detector 2040 as discussed below. A scanning transmission electron microscopy (STEM) detector 2062, located beneath a transmission electron microscopy (TEM) sample holder 2024 and the movable X-Y stage 2025, can collect electrons that are transmitted through the sample mounted on the TEM sample holder 2024 as discussed above.

The dual beam system 2010 can also include a focused ion beam (FIB) system 2011 which can comprise an evacuated chamber having an upper neck portion 2012 within which can be located an ion source 2014 and a focusing column 2016 including extractor electrodes and an electrostatic optical system. The axis of the focusing column 2016 can be tilted 52 degrees (or any other suitable angular displacement) from the axis of the electron column. The ion column 2012 can include an ion source 2014, an extraction electrode 2015, a focusing element 2017, deflection elements 2020, and a focused ion beam 2018. The focused ion beam 2018 can pass from the ion source 2014 through the focusing column 2016 and between electrostatic deflection means schematically indicated at numeral 2020 toward the substrate 2022, which can comprise, for example, a semiconductor device positioned on the movable X-Y stage 2025 within the lower chamber 2026.

The movable X-Y stage 2025 can move in a horizontal plane (along X and Y axes) and vertically (along Z axis). The movable X-Y stage 2025 can tilt approximately sixty (60) degrees and rotate about the Z axis. In some embodiments, a separate TEM sample stage (not shown) can be used. Such a TEM sample stage can be moveable in the X, Y, and Z axes. A door 2061 can be opened for inserting the substrate 2022 onto the movable X-Y stage 2025 or also for servicing an internal gas supply reservoir, if one is used. The door 2061 can be interlocked so that it cannot be opened if the system is under vacuum.

An ion pump 2068 can be employed for evacuating the neck portion 2012. The chamber 2026 can be evacuated with a turbomolecular and mechanical pumping system 2030 under the control of a vacuum controller 2032. Such vacuum system can provide within the chamber 2026 a vacuum of between approximately 1 x 10⁻⁷ Torr and 5 x 10⁻⁴ Torr. If an etch assisting, an etch retarding gas, or a deposition precursor gas is used, the chamber background pressure may rise, typically to about 1 x 10⁻⁵ Torr.

A high voltage power supply 2034 can provide an appropriate acceleration voltage to electrodes in the focusing column 2016 for energizing and the focused ion beam 2018. When it strikes the substrate 2022, material can be sputtered (that is, physically ejected) from the sample. Alternatively, the focused ion beam 2018 can decompose a precursor gas to deposit a material.

The high voltage power supply 2034 can be connected to the ion source 2014 (which can be a liquid metal ion source) as well as to appropriate electrodes in the ion beam focusing column 2016 for forming an approximately 1 keV to 60 keV ion beam 2018 and directing the same toward a sample. A deflection controller and amplifier 2036, operated in accordance with a prescribed pattern provided by a pattern generator 2038, can be coupled to the deflection elements 2020 (which can be deflection plates) whereby the focused ion beam 2018 may be controlled manually or automatically to trace out a corresponding pattern on the upper surface of the substrate 2022. In some systems, the deflection elements 2020 can be placed before the final lens. Beam blanking electrodes (not shown) within the ion beam focusing column 2016 can cause the focused ion beam 2018 to impact onto a blanking aperture (not shown) instead of the substrate 2022 when a blanking controller (not shown) applies a blanking voltage to a blanking electrode.

The ion source 2014 can provide a metal ion beam of gallium, for example. In other examples, the ion source 2014 may be a plasma ion source that extracts ions from a generated plasma. The source can be capable of being focused into a sub one-tenth micrometer wide beam at the substrate 2022 for either modifying the substrate 2022 by ion milling, enhanced etch, material deposition, or for the purpose of imaging the substrate 2022.

A charged particle detector 2040, such as an Everhart Thornley or multichannel plate, used for detecting secondary ion or electron emission can be connected to a video circuit 2042 that can supply drive signals to a video monitor 2044 and receive deflection signals from a system controller 2019. The location of the charged particle detector 2040 within the lower chamber 2026 can vary in different embodiments. For example, the charged particle detector 2040 can be coaxial with the ion beam and include a hole for allowing the ion beam to pass. In other embodiments, secondary particles can be collected through a final lens and then diverted off axis for collection.

A micromanipulator 2047 can precisely move objects within the vacuum chamber. The micromanipulator 2047 may comprise precision electric motors 2048 positioned outside the vacuum chamber to provide X, Y, Z, and theta control of a portion 2049 positioned within the vacuum chamber. The micromanipulator 2047 can be fitted with different end effectors for manipulating small objects. In various embodiments described herein, the end effector can be a thin probe 2050.

A gas delivery system 2046 can extend into the lower chamber 2026 for introducing and directing a gaseous vapor toward the substrate 2022. U.S. Pat. No. 5,851,413 to Casella et al. for "Gas Delivery Systems for Particle Beam Processing," assigned to the assignee of the present invention, describes a suitable gas delivery system 2046. Another gas delivery system is described in U.S. Pat. No. 5,435,850 to Rasmussen for a "Gas Injection System," also assigned to the assignee of the present invention. For example, iodine can be delivered to enhance etching, or a metal organic compound can be delivered to deposit a metal.

The system controller 2019 can control the operations of the various parts of the dual beam system 2010. Through the system controller 2019, a user can cause the focused ion beam 2018 or the electron beam 2043 to be scanned in a desired manner through commands entered into any suitable user interface (not shown). Alternatively, the system controller 2019 may control the dual beam system 2010 in accordance with programmed instructions stored in a memory 2021. In various embodiments, any of the one or more software components 319 can be implemented in or otherwise executed by the system controller 2019.

Various embodiments may be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

Various non-limiting aspects are described in the following examples.

EXAMPLE 1: A system can comprise: a processor that executes computer-executable components stored in a non-transitory computer-readable memory, wherein the computer-executable components can comprise: a workflow component that can cause a charged-particle microscope to perform a workflow on a specimen; a status component that, in response to the charged-particle microscope generating an error message that interrupts the workflow, can retrieve runtime data logged by the charged-particle microscope during the workflow; and a model component that can synthesize, via execution of a large language model on the error message and on the runtime data, first text that explains why the workflow was interrupted.

EXAMPLE 2: The system of any preceding example can be implemented, wherein the model component can further synthesize, via execution of the large language model on the first text, second text that explains how to resolve the workflow.

EXAMPLE 3: The system of any preceding example can be implemented, wherein the model component can further synthesize, via execution of the large language model on the second text, a coding script that is configured to cause the charged-particle microscope to resolve the workflow.

EXAMPLE 4: The system of any preceding example can be implemented, wherein the computer-executable components further comprise: an execution component that can cause the charged-particle microscope to execute the coding script, thereby resolving the workflow.

EXAMPLE 5: The system of any preceding example can be implemented, wherein the workflow can comprise: an imaging scan of the specimen; a milling operation of the specimen; or a movement of an actuatable stage holding the specimen.

EXAMPLE 6: The system of any preceding example can be implemented, wherein the runtime data can comprise: a partial image of the specimen captured by the charged-particle microscope during the workflow; or states that one or more configurable operating parameters of the charged-particle microscope had during the workflow.

EXAMPLE 7: The system of any preceding example can be implemented, where the large language model can synthesize the first text in retrieval-augmented generative fashion based on performing an embedding search through a repository of technical documentation associated with a design, manufacture, operation, or troubleshooting of the charged-particle microscope, and wherein the computer-executable components can further comprise: an execution component that can receive feedback from a user or technician regarding the first text, wherein the model component can synthesize, via execution of the large language model on the feedback, a textual edit to a document in the repository of technical documentation that was retrieved by the embedding search, and wherein the execution component can update the repository of technical documentation by inserting the textual edit into the document.

EXAMPLE 8: The system of any preceding example can be implemented, wherein the computer-executable components can further comprise: an execution component that can visually or audibly render the first text on an electronic screen or an electronic speaker.

In various embodiments, any combination or combinations of examples 1-8 can be implemented.

EXAMPLE 9: A computer-implemented method can comprise: causing, by a device operatively coupled to a processor, a charged-particle microscope to perform a workflow on a specimen; retrieving, by the device and in response to the charged-particle microscope generating an error message that interrupts the workflow, runtime data logged by the charged-particle microscope during the workflow; and synthesizing, by the device and via execution of a large language model on the error message and on the runtime data, first text that explains why the workflow was interrupted.

EXAMPLE 10: The computer-implemented method of any preceding example can be implemented, further comprising: synthesizing, by the device and via execution of the large language model on the first text, second text that explains how to resolve the workflow.

EXAMPLE 11: The computer-implemented method of any preceding example can be implemented, further comprising: synthesizing, by the device and via execution of the large language model on the second text, a coding script that is configured to cause the charged-particle microscope to resolve the workflow.

EXAMPLE 12: The computer-implemented method of any preceding example can be implemented, further comprising: causing, by the device, the charged-particle microscope to execute the coding script, thereby resolving the workflow.

EXAMPLE 13: The computer-implemented method of any preceding example can be implemented, wherein the workflow can comprise: an imaging scan of the specimen; a milling operation of the specimen; or a movement of an actuatable stage holding the specimen.

EXAMPLE 14: The computer-implemented method of any preceding example can be implemented, wherein the runtime data can comprise: a partial image of the specimen captured by the charged-particle microscope during the workflow; or states that one or more configurable operating parameters of the charged-particle microscope had during the workflow.

EXAMPLE 15: The computer-implemented method of any preceding example can be implemented, wherein the large language model can synthesize the first text in retrieval-augmented generative fashion based on performing an embedding search through a repository of technical documentation associated with a design, manufacture, operation, or troubleshooting of the charged-particle microscope, and further comprising: receiving, by the device, feedback from a user or technician regarding the first text; synthesizing, by the device and via execution of the large language model on the feedback, a textual edit to a document in the repository of technical documentation that was retrieved by the embedding search; and updating, by the device, the repository of technical documentation by inserting the textual edit into the document.

EXAMPLE 16: The computer-implemented method of any preceding example can be implemented, further comprising: visually or audibly rendering, by the device, the first text on an electronic screen or an electronic speaker.

In various embodiments, any combination or combinations of examples 9-16 can be implemented.

EXAMPLE 17: A computer program product for facilitating large language model resolution of scientific instrument workflow interruptions can comprise a non-transitory computer-readable memory having program instructions embodied therewith. In various aspects, the program instructions can be executable by a processor to cause the processor to: cause a scientific instrument to perform a workflow on a specimen; retrieve, in response to the scientific instrument generating an error message that interrupts the workflow, runtime data logged by the scientific instrument during the workflow; and synthesize, via execution of a large language model on the error message and on the runtime data, first text that explains why the workflow was interrupted.

EXAMPLE 18: The computer program product of any preceding example can be implemented, wherein the program instructions can be further executable to cause the processor to: synthesize, via execution of the large language model on the first text, second text that explains how to resolve the workflow.

EXAMPLE 19: The computer program product of any preceding example can be implemented, wherein the program instructions can be further executable to cause the processor to: synthesize, via execution of the large language model on the second text, a coding script that is configured to cause the scientific instrument to resolve the workflow; and cause the scientific instrument to execute the coding script, thereby resolving the workflow.

EXAMPLE 20: The computer program product of any preceding example can be implemented, wherein the scientific instrument can be a charged-particle microscope, a chromatograph, or a mass spectrometer.

In various embodiments, any combination or combinations of examples 17-20 can be implemented.

In various embodiments, any combination or combinations of examples 1-20 can be implemented.

## Claims

1. A system, comprising:
a processor that executes computer-executable components stored in a non-transitory computer-readable memory, wherein the computer-executable components comprise:
a workflow component that causes a charged-particle microscope to perform a workflow on a specimen;
a status component that, in response to the charged-particle microscope generating an error message that interrupts the workflow, retrieves runtime data logged by the charged-particle microscope during the workflow; and
a model component that synthesizes, via execution of a large language model on the error message and on the runtime data, first text that explains why the workflow was interrupted.

2. The system of claim 1, wherein the model component further synthesizes, via execution of the large language model on the first text, second text that explains how to resolve the workflow.

3. The system of claim 2, wherein the model component further synthesizes, via execution of the large language model on the second text, a coding script that is configured to cause the charged-particle microscope to resolve the workflow.

4. The system of claim 3, wherein the computer-executable components further comprise:
an execution component that causes the charged-particle microscope to execute the coding script, thereby resolving the workflow.

5. The system of claim 1, wherein the workflow comprises: an imaging scan of the specimen; a milling operation of the specimen; or a movement of an actuatable stage holding the specimen.

6. The system of claim 1, wherein the runtime data comprises: a partial image of the specimen captured by the charged-particle microscope during the workflow; or states that one or more configurable operating parameters of the charged-particle microscope had during the workflow.

7. The system of claim 1, where the large language model synthesizes the first text in retrieval-augmented generative fashion based on performing an embedding search through a repository of technical documentation associated with a design, manufacture, operation, or troubleshooting of the charged-particle microscope, and wherein the computer-executable components further comprise:
an execution component that receives feedback from a user or technician regarding the first text, wherein the model component synthesizes, via execution of the large language model on the feedback, a textual edit to a document in the repository of technical documentation that was retrieved by the embedding search, and wherein the execution component updates the repository of technical documentation by inserting the textual edit into the document.

8. The system of claim 1, wherein the computer-executable components further comprise:
an execution component that visually or audibly renders the first text on an electronic screen or an electronic speaker.

9. A computer-implemented method, comprising:
causing, by a device operatively coupled to a processor, a charged-particle microscope to perform a workflow on a specimen;
retrieving, by the device and in response to the charged-particle microscope generating an error message that interrupts the workflow, runtime data logged by the charged-particle microscope during the workflow; and
synthesizing, by the device and via execution of a large language model on the error message and on the runtime data, first text that explains why the workflow was interrupted.

10. The computer-implemented method of claim 9, further comprising:
synthesizing, by the device and via execution of the large language model on the first text, second text that explains how to resolve the workflow; and.
synthesizing, by the device and via execution of the large language model on the second text, a coding script that is configured to cause the charged-particle microscope to resolve the workflow.

11. The computer-implemented method of claim 10, further comprising:
causing, by the device, the charged-particle microscope to execute the coding script, thereby resolving the workflow.

12. The computer-implemented method of claim 9, wherein the workflow comprises: an imaging scan of the specimen; a milling operation of the specimen; or a movement of an actuatable stage holding the specimen.

13. The computer-implemented method of claim 9, wherein the runtime data comprises: a partial image of the specimen captured by the charged-particle microscope during the workflow; or states that one or more configurable operating parameters of the charged-particle microscope had during the workflow.

14. The computer-implemented method of claim 9, where the large language model synthesizes the first text in retrieval-augmented generative fashion based on performing an embedding search through a repository of technical documentation associated with a design, manufacture, operation, or troubleshooting of the charged-particle microscope, and further comprising:
receiving, by the device, feedback from a user or technician regarding the first text;
synthesizing, by the device and via execution of the large language model on the feedback, a textual edit to a document in the repository of technical documentation that was retrieved by the embedding search; and
updating, by the device, the repository of technical documentation by inserting the textual edit into the document.

15. The computer-implemented method of claim 9, further comprising:
visually or audibly rendering, by the device, the first text on an electronic screen or an electronic speaker.
